# EUROPEAN PATENT APPLICATION

(11) **EP 4 342 988 A1**
(43) Date of publication of application: **27.03.2024**
(21) Application number: 22196721.9
(22) Date of filing: 20.09.2022
(51) Int. Cl.: C12N 15/113, C12N 9/22, A61P 25/28

(54) **COMPOSITIONS AND METHODS FOR MODIFICATION OF THE EXPRESSION OF A SNCA GENE**

(71) Applicant: Eberhard Karls Universität Tübingen, Medizinische Fakultät, 72074 Tübingen (DE)
(72) Inventor: Cheng, Fubo, 72076 Tübingen (DE); Rieß, Olaf, 72070 Tübingen (DE)
(74) Representative: Witte, Weller & Partner Patentanwälte mbB

(57) **Abstract**

The present invention is directed to a composition for modification of the expression of a *SNCA* gene, an isolated polynucleotide encoding said composition, a vector comprising the isolated polynucleotide, a host cell comprising the isolated polynucleotide or the vector, a pharmaceutical composition comprising at least one of the composition, the isolated polynucleotide, the vector, or the host cell, a kit comprising at least one of the composition, the isolated polynucleotide, the vector, or the host cell, a method of modulation of expression of the SNCA gene in a cell or a subject, and to a method of treating a disease or disorder associated with altered, preferably elevated SNCA gene expression levels in a subject.

## Description

The present invention is directed to a composition for modification of the expression of a *SNCA* gene, an isolated polynucleotide encoding said composition, a vector comprising the isolated polynucleotide, a host cell comprising the isolated polynucleotide or the vector, a pharmaceutical composition comprising at least one of the composition, the isolated polynucleotide, the vector, or the host cell, a kit comprising at least one of the composition, the isolated polynucleotide, the vector, or the host cell, a method of modulation of expression of the SNCA gene in a cell or a subject, and to a method of treating a disease or disorder associated with altered, preferably elevated SNCA gene expression levels in a subject.

### FIELD OF THE INVENTION

The present invention relates to the field of molecular medicine, more particularly to the field of genetic engineering applications, preferably to the targeted modification of the expression of disease-associated genes.

### BACKGROUND OF THE INVENTION

Parkinson's disease (PD) is one of the most common neurodegenerative diseases. There is no effective treatment to prevent PD or to halt its progression. In PD alpha-synuclein, encoded by the *SNCA* gene, is a major component of Lewy bodies (LBs). Although single point mutations in the *SNCA* gene can cause early-onset familial forms of autosomal dominant inherited PD, several lines of evidence support that increased dosage of wild-type human SNCA protein level is critical to PD pathogenesis as well. Duplication, triplication, or rearrangements of the wild-type *SNCA* gene cause familial PD, and the severity of clinical phenotypes correlates with the number of *SNCA* copies. Furthermore, polymorphisms in *SNCA* gene regulatory regions which enhance *SNCA* expression are associated with sporadic PD. Similarly, mouse and rat models overexpressing human wild-type alpha-synuclein protein showed alpha-synucleinopathies, nigrostriatal degeneration, and even inclusion body formation. In addition, elevated levels of *SNCA* transcripts were detected in PD post-mortem brains.

Therefore, to date, *SNCA* is one of the most validated and promising therapeutic targets for PD, and manipulations of SNCA levels have demonstrated a beneficial impact.

In the WO 2006/039253 methods using RNA interference (RNAi) tools are disclosed to directly target SNCA transcripts as a therapeutic approach to treat neurodegenerative diseases such as PD.

In the WO 2019/209869 methods are proposed for the treatment of PD via the downregulation of SNCA expression by Cas9-based editing of DNA methylation. In the WO 2018/154418 methods using CRISPR-targeted destroying of an 3'-end enhancer of the *SNCA* gene for the treatment of early onset PD are described.

However, the known methods have not proved successful in practice, especially as they are associated with a number of disadvantages and, above all, side effects. This is mainly due to the fact that in both procedures the expression of *SNCA* is not only affected in neuronal tissues, but also in non-neuronal tissues, i.e., outside of the brain. Furthermore, the RNAi approach bears two significant shortcomings. First, RNAi does not provide a fine resolution for the knockdown where a tight regulation is desired to achieve "physiological" level of SNCA expression. For example, AAV-vector harboring siRNA against SNCA-mRNA showed high-levels of toxicity and caused a significant loss of nigrostriatal dopaminergic neurons, because of robust reduction of SNCA levels as demonstrated in rat models. Consistently, downregulation of SNCA in MN9D cells decreased cell viability. Second, RNAi can affect the expression of genes other than their intended targets, as demonstrated by whole genome expression profiling after siRNA transfection.

Studies in animals or humans for the treatment of PD and other neuro-generative disorders involving the administration of siRNA or shRNA with the aim to reduce the level of endogenous SNCA are summarized in Nakamori et al. (2019), Nucleic acid-based therapeutics for Parkinson's Disease. Neurotherapeutics 16(2):287-298.

Antisense oligonucleotides (ASO) like Amido-bridged nucleic acid (AmNA)-modified antisense oligonucleotides were also implicated as potential treatment targeting the *SNCA* gene; Uehara et al. (2019), Amido-bridged nucleic acid (AmNA)-modified antisense oligonucleotides targeting α-synuclein as a novel therapy for Parkinson's disease. Sci. Rep. 9(1):7567.

Although a reduction of the SNCA expression through different methods can protect neurodegeneration induced by hSNCA (Khodr et al. (2011), An α-synuclein AAV gene silencing vector ameliorates a behavioral deficit in a rat model of Parkinson's disease, but displays toxicity in dopamine neurons. Brain Res. 1395:94-107), rotenone (Zharikov et al. (2015), shRNA targeting α-synuclein prevents neurodegeneration in a Parkinson's disease model. J. Clin. Invest. 125(7):2721-35), or 1-methyl-4-phenyl-1,2,3,6-tetrahydropyridine (MPTP) (Javed et al. (2016), Development of nonviral vectors targeting the brain as a therapeutic approach for Parkinson's Disease and other brain disorders. Mol. Ther. 2016 Apr;24(4):746-58), side effects like toxicity or inflammation caused by vector or injection itself and reduced dopamine content and TH neurons due to the extremely lower level of endogenous SNCA were also observed (Khodr et al., *loc. cit.*; Zharikov et al., *loc. cit.*; Javed et al., *loc. cit*.).

Similarly, targeting general transcription factors or modifying histone modification in general, like H3K27ac (Mittal et al. (2017), β2-Adrenoreceptor is a regulator of the α-synuclein gene driving risk of Parkinson's disease. Science 357(6354):891-898), might not be an ideal treatment strategy for PD, as both strategies will cause global gene expression changes and subsequently may cause unknown side effects. Using CRISPR/dCas9 based technique to modify the DNA methylation or histone modification on the SNCA promoter have been reported as a treatment through which the SNCA expression in culture neurons was down-regulated (Kantor et al. (2018), Downregulation of SNCA expression by targeted editing of DNA methylation: A potential strategy for precision therapy in PD. Mol. Ther. 26(11):2638-2649 and Guhathakurta et al. (2021), Targeted attenuation of elevated histone marks at SNCA alleviates α-synuclein in Parkinson's disease. EMBO Mol Med. 13(2):e12188). However, unwanted *in vivo* and long-term treatment effects of these virus vector based treatments are to be expected.

Therefore, the identification and validation of a target for achieving tight regulation of *SNCA* transcription that will allow maintaining normal physiological levels of alpha-synuclein specifically in the brain is needed.

The role of *SNCA* overexpression in PD pathogenesis on the one hand, and the need to maintain normal physiological levels of alpha-synuclein protein in the brain on the other, emphasize the so-far unmet need to develop new therapeutic strategies targeting the regulatory mechanisms of SNCA expression in neuronal tissues. Thus, the object underlying the invention is to provide new therapeutic strategies targeting the regulation of *SNCA* expression in the brain.

The present invention satisfies these and other needs.

### SUMMARY OF THE INVENTION

The present invention provides a composition for the modification of the expression of a *SNCA* gene, the composition comprising:
(a) at least one nucleic acid molecule comprising a nucleotide sequence substantially complementary to the nucleotide sequence of a target region within an intronic enhancer of the *SNCA* gene located within intron 4 of the *SNCA* gene.

According to the invention "a composition" includes both an assembly of the nucleic acid molecule together with other components, such as protein(s), another nucleic acid molecule(s), and other substance(s), or solely the nucleic acid molecule according to the invention.

According to the invention, the "*SNCA* gene" comprises one from any living being having this gene, such as a mammal, in particular a human being.

According to the invention, "modification" includes both "increasing" and, preferably, "reducing" the expression of a *SNCA* gene, respectively. As used herein, the terms "increasing", "increased", "increase" (and grammatical variations thereof) describe an elevation of at least about 25%, 50%, 75%, 100%, 150%, 200%, 300%, 400%, 500% or more as compared to a control. To the contrary, the terms "reduction", "reduce", "reduced", and "reducing", (and grammatical variations thereof), describe, for example, a decrease of at least about 5%, 10%, 15%, 20%, 25%, 35%, 50%, 75%, 80%, 85%, 90%, 95%, 97%, 98%, 99%, or 100% as compared to a control, such as the expression level of the *SNCA* gene in a reference subject not treated with the composition according to the invention. In particular embodiments, the reduction results in no or essentially no (i.e., an insignificant amount, e.g., less than about 10% or even less than about 5%) detectable expression.

"Nucleic acid" or, synonymously, "oligonucleotide" or "polynucleotide" as used herein means at least two nucleotides covalently linked together. The nucleic acid molecule according to the invention may be preferably single stranded, but in a specific embodiment double stranded, or may contain portions of both double stranded and single stranded sequence. The nucleic acid may be DNA, both genomic and cDNA, RNA, or a hybrid, where the nucleic acid may contain combinations of deoxyribo- and ribonucleotides, and combinations of bases including uracil, adenine, thymine, cytosine, guanine, inosine, xanthine hypoxanthine, isocytosine and isoguanine. Nucleic acids may be obtained by chemical synthesis methods or by recombinant methods. Except as otherwise indicated, nucleic acid molecules and/or polynucleotides provided herein are presented herein in the 5' to 3' direction, from left to right and are represented using the standard code for representing the nucleotide characters.

"Complementary" or "complement" or as used herein refers to a nucleotide sequence established in view of the reference nucleotide sequence, i.e., of the target region, according to Watson-Crick (e.g., A-T/U and C-G) or Hoogsteen base pairing rules. "Complementarity" refers to a property shared between the nucleotide sequence of the nucleic acid of the composition and the nucleotides sequence of the target region, such that when they are aligned antiparallel to each other, the nucleotide bases at each position will be complementary. "Complement" as used herein can mean 100% complementarity (fully complementary) with the target nucleotide sequence or, in embodiments of the invention, it can mean less than 100% complementarity (e.g., substantial complementarity) (e.g., about 80%, 81%, 82%, 83%, 84%, 85%, 86%, 87%, 88%, 89%, 90%, 91%, 92%, 93%, 94%, 95%, 96%, 97%, 98%, 99%, and the like, complementarity). The complementary sequences hybridize to each other under stringent conditions.

Due to the complementarity of the nucleotide sequence of the nucleic acid molecule of the invention, the latter can be a classical antisense oligonucleotide (ASO) but also guide RNA (gRNA) or comparable.

As used herein, the term "*SNCA* gene" refers to a nucleic acid molecule capable of being used to produce mRNA and may or may not be capable of being used to produce the functional alpha-synuclein or *SNCA* gene product. The *SNCA* gene includes both coding (i.e., exons) and non-coding regions (e.g., introns, regulatory elements, promoters, enhancers, termination sequences and/or 5' and 3' untranslated regions). The gene can be in its natural environment or can be "isolated" by which is meant a nucleic acid that is substantially or essentially free from components normally found in association with the nucleic acid in its natural state. Such components include other cellular material, culture medium from recombinant production, and/or various chemicals used in chemically synthesizing the nucleic acid.

"Enhancer" as used herein means a synthetic or naturally derived molecule which can be bound by proteins (e.g., activators) to increase the likelihood that transcription of a particular gene, e.g., the *SNCA* gene, will occur. These proteins or activators are usually referred to as transcription factors. Enhancers are *cis*-acting. They can be located up to 1 Mbp (1,000,000 bp) away from the gene, upstream or downstream from the start site.

"Target region" as used herein refers to the region of the enhancer of the target SNCA gene to which the composition according to the invention is designed to bind and modify.

According to the invention, an "intronic enhancer" is understood to be such an enhancer that is located between coding segments, i.e., exons, in the non-coding region, i.e., introns, usually, but not exclusively, on the DNA of the genome or copies or transcripts thereof.

According to the invention, the enhancer(s) is/are located (in the human genome) in intron 4, between exon 4 and exon 5.

The inventors surprisingly found that the expression of the *SNCA* gene in the brain is predominantly controlled by intronic enhancer(s) located within intron 4 of the *SNCA* gene while such enhancer-controlled *SNCA* gene expression cannot be found or only to a lesser extent in other tissues. This tissue-restricted expression control opens a specific therapeutic option directed to neurological disorders with limited or even the lack of effects on non-neuronal tissues. Such novel therapeutic approach is, therefore, safe and poor in side effects. The inventors were able to demonstrate impressively that this therapeutic approach works by using an animal model that is well established among experts. These advantages, in particular the low side-effect profile of the invention, are in marked contrast to the disadvantageous methods in the prior art, as described, for example, in the WO 2018/154418, WO 2019/209869 and WO 2006/039253 (*loc. cit*.).

In an embodiment of the invention said at least one nucleic acid molecule is
(i) a guide RNA (gRNA), or/and
(ii) a nucleic acid molecule encoding said at least one gRNA.

This measure advantageously sets the stage for the use of the invention in the CRISPR/Cas system. A guide RNA (gRNA; also referred interchangeably herein as "single guide RNA" or "sgRNA") is a non-coding short RNA sequence which binds to the complementary target DNA sequence. It functions as a guide for RNA- or DNA-targeting enzymes, such as type I and I Cas proteins, with which it forms complexes. Typically, these enzymes will delete, insert or otherwise alter the targeted RNA or DNA, i.e., target region within an enhancer of the *SNCA* gene.

According to the invention "a nucleic acid molecule encoding at least one gRNA" refers to a DNA, or cDNA or mRNA, which comprise or consist of the coding information for the synthesis of the gRNA.

In still another embodiment of the invention the composition further comprises
(b)
(i) a Clustered Regulatory Interspaced Short Palindomic Repeats associated (Cas) protein, or/and
(ii) a nucleic acid molecule encoding said Cas protein,
wherein said gRNA is configured to guide the Cas protein to a target region within an enhancer of the *SNCA* gene.

With this measure, the composition according to the invention is supplemented with the Cas protein or a nucleic acid molecule encoding it and can thus be used without further ado in an advantageous manner as a gene editing tool.

According to the invention "a nucleic acid molecule encoding the Cas protein" refers to a DNA, or cDNA or mRNA, which comprise or consist of the coding information for the synthesis of the Cas protein.

In another embodiment of the invention said at least one nucleic acid molecule is an antisense oligonucleotide (ASO) against enhancer RNA (eRNA).

This measure has the advantage that a recently discover functional molecule derived from the enhancer is addressed for modifying the *SNCA* gene expression. Therefore, the eRNA is an intron 4 enhancer(s) derived eRNA, i.e., an eRNA that is transcribed from the intronic enhancer(s) located in intron 4 of the *SNCA* gene.

Enhancer RNAs (eRNAs) represent a class of relatively long non-coding RNA molecules (50-2000 nucleotides) transcribed from the DNA sequence of enhancer regions. They were first detected in 2010 through the use of genome-wide techniques such as RNA-seq and ChIP-seq. The expression of the eRNA correlates with the activity of its corresponding enhancer in the target genes, i.e., *SNCA.* eRNAs actively play a role in transcriptional regulation in *cis* and in *trans*, and while their mechanisms of action remain unclear.

In still a preferred embodiment, it is a human *SNCA* gene (*hSNCA*) (Gene ID: 6622), the expression of which can be specifically modified by the composition according to the invention.

In yet another embodiment of the invention the enhancer is located at Chr4: 90720577-90722136 (En-1) and/or at Chr4: 90673800-90675200 (En-2), and, preferably, comprises the nucleotide sequence of SEQ ID NO: 1 (En-1) and/or SEQ ID NO: 2 (En-2).

This measure advantageously establishes the design conditions for a nucleic acid molecule that is highly complementary with respect to the *SNCA* gene intronic enhancer(s), since the specific nucleotide sequences resulting from the positional information of the enhancers enable precise fabrication. In particular, the inventors were able to find two intronic enhancers (En-1 and En-2), both located on human chromosome 4, that are especially suitable according to the invention. The enhancer "En-1" is generally known from McClymont et al. (2018), Parkinson-associated SNCA enhancer variants revealed by open chromatin in mouse dopamine neurons. Am. J. Hum. Genet. 103(6):874-892.

In another embodiment of the invention the at least one gRNA of the composition comprises a nucleotide sequence of at least one of SEQ ID NO: 3 (gRNA1F), SEQ ID NO: 4 (gRNA1R), SEQ ID NO: 5 (gRNA2F), SEQ ID NO: 6 (gRNA2R), SEQ ID NO: 7 (gRNAtF), SEQ ID NO: 8 (gRNAtR).

As a convention, it is understood that all nucleotide sequences of the present disclosure are given in terms of DNA sequences. However, the sequences also include RNA sequences. The RNA sequence corresponding to a specified DNA sequence results from the fact that all thymines (T, t) are replaced by uracils (U, u). Such an exchange takes place in particular when reference is made to gRNA molecules, but a DNA sequence is explicitly specified. This means, for example, that the nucleotide sequence SEQ ID NO: 3: GGGCGAGAATAGTCAATATC specified in the form of DNA comprises the following RNA nucleotide sequence: GGGCGAGAAUAGUCAAUAUC. The same applies to the nucleotide sequences of the remaining gRNA molecules according to the invention.

In this advantageous embodiment of the composition according to the invention, gRNA molecules or sequences are used which, according to the knowledge of the inventors, have proved particularly suitable for brain specific *SNCA* gene expression modification. Preferably, a pair of gRNA molecules is always used as follows, where the numbers 1, 2, and 3 stand for the respective pair, and "F" for "forward" and "R" for "reverse":
1: SEQ ID NO: 3 (gRNA1F), SEQ ID NO: 4 (gRNA1R), and/or
2: SEQ ID NO: 5 (gRNA2F), SEQ ID NO: 6 (gRNA2R), and/or
3: SEQ ID NO: 7 (gRNAtF), SEQ ID NO: 8 (gRNAtR).

Pair 1 interacts with the intronic enhancer 1 and pair 2 interacts with the intronic enhancer 2. The gRNA pair 3 includes two gRNAs, one locates upstream of enhancer 1 and the other one locates on downstream of enhancer 2, which pair can therefore be used to modify a large intronic region that includes intronic enhancers 1 and 2.

In a further embodiment of the invention the Cas protein is a Cas9 protein, preferably a SpCas9 protein.

This measure has the advantage that such a CRISPR endonuclease is used, which is well established and characterized in the art and properly suited for carrying out the invention. Cas9, formerly also called Cas5, Csn1, or Csx12, is a 160 kilodalton protein. It originates from the Gram-positive, aerotolerant bacterium *Streptococcus pyogenes* where it plays a vital role in the immunological defense against DNA viruses and plasmids. Cas9 can cleave nearly any sequence complementary to the guide RNA. Because the target specificity of Cas9 stems from the guide RNA:DNA complementarity and not from modifications to the protein itself, engineering Cas9 to target new DNA is straightforward. This embodiment allows a targeted knocking-out or inactivation of the intronic enhancer regions by causing double or single strand breaks in the target region, thereby modifying or reducing the expression of the *SNCA* gene in the brain.

In another embodiment of the composition of the invention the Cas protein is a dead Cas9 (dCas9) protein, preferably a dCas9 fused to a protein domain exhibiting histone-deacetylation activity, further preferably dCas9 fused to HDAC3 protein and/or a peptide derived from Krüppel associated box (KRAB) domain.

This measure has the advantage that the modification of the expression of the *SNCA* gene is achieved by means of using so-called CRISPR-dCas9-based artificial transcription factors (ATFs); see Martinez-Escobar et al. (2021), CRISPR-dCas9-based artificial transcription factors to improve efficacy of cancer treatment with drug re-purposing: proposal for future research. Front. Oncol. 10:604948. The "dead" or defective Cas9 (dCas9) has lost endonuclease activity and, therefore, its ability to cut nucleic acid or DNA, respectively. However, it functions as an ATF due to its capability to manipulate the expression of the *SNCA* gene. In contrast to using functional Cas9 this approach has the advantage that there is no risk of off-target genomic editing and permanent DNA alterations with eventual disruption of gene function; see Ricci and Colasante (2021), CRISPR/dCas9 as a therapeutic approach for neurodevelopmental disorders: innovations and limitations compared to traditional strategies. Dev. Neurosci. 43:253-261. With the help of gRNA, the dCas9 protein can move to the target enhancer region, which will, however, not cause a DNA break. In the preferred embodiment the protein domain exhibiting histone-deacetylation activity, preferably a HDAC3 protein, can lead to de-acetylation of the histone, for example de-acetylation of the H3K27ac (an epigenetic modification to the DNA packaging protein histone H3 which is a marker for enhancer activity), and finally represses the intronic enhancer activities. Another marker for enhancer activity is a peptide derived from Krüppel associated box (KRAB) domain.

In another embodiment of the composition according to the invention the nucleic acid of (a)(ii) and/or (b)(ii) comprises DNA or RNA.

In yet a further embodiment in the composition according to the invention one or both of (a) and (b) are packaged in a vector, preferably a viral vector.

"Vector" as used herein means a nucleic acid sequence containing an origin of replication. A vector can be, preferably, a viral vector, or a bacteriophage, bacterial artificial chromosome or yeast artificial chromosome. A vector can be a DNA or RNA vector. A vector can be a self-replicating extrachromosomal vector, and further preferably, is a DNA plasmid.

Another subject-matter of the invention relates to said composition for use in the treatment of a neurological disease, preferably neurodegenerative diseases. The neurodegenerative diseases are further preferably selected form the group consisting of: synucleopathies, including α-synucleopathy, *SNCA*-associated neurodegenerative diseases such as multiple system atrophy 1 (MSA1), Parkinson's disease (PD), and dementia with Lewy bodies.

The inventors have found that the composition according to the invention interferes with the molecular mechanisms involved in the pathogenesis of said neurological diseases or disorders. This subject-matter, therefore, allows for the treatment of such neurological afflictions for which current treatment approaches have so far failed to provide satisfactory remedies.

A still other subject-matter of the invention is an isolated polynucleotide encoding the composition according to the invention. Further, another subject-matter of the invention is a vector, preferably a viral vector, comprising said isolated polynucleotide Another subject-matter of the invention relates to a host cell comprising said isolated polynucleotide or vector.

An "isolated" polynucleotide is a nucleotide sequence that, by the hand of man, exists apart from its native environment and is therefore not a product of nature. In this embodiment, the polynucleotide is "isolated". An isolated polynucleotide can exist in a purified form that is at least partially separated from at least some of the other components of the naturally occurring organism or virus, for example, the cell or viral structural components or other polypeptides or polynucleotides commonly found associated with the polypeptide or polynucleotide. In representative embodiments, the isolated is at least about 1%, 5%, 10%, 20%, 30%, 40%, 50%, 60%, 70%, 80%, 90%, 95%, or more pure.

Host cells include a myriad of cell types, for example, including, but not limited to eukaryotic cells or prokaryotic cells. In some embodiments, the eukaryotic cell can be any eukaryotic cell from any eukaryotic organism. Non-limiting examples of eukaryotic organisms include mammals, insects, amphibians, reptiles, birds, fish, fungi, plants, and/or nematodes. In some embodiments, the cell is a mammalian cell. In some embodiments, the cell is a human cell. In some embodiments, the cell is a neuronal cell. For example, the cell may be a midbrain dopaminergic neuron (mDA). The cell may be a basal forebrain cholinergic neuron (BFCN). In other embodiments, the cell may be a neural progenitor cell. For example, the cell may be a dopaminergic (ventral midbrain) Neural Progenitor Cell (MD NPC). The cell may comprise a mutation in the *SNCA* gene. For example, the cell may comprise a mutation in the *SNCA* gene that causes increased *SNCA* gene expression in the cell or subject. In some embodiments, the cell may comprise a *SNCA* gene triplication (*SNCA*-Tri), wherein the levels of *SNCA* are elevated compared to physiological levels in a control cell that does not have **SNCA**-Tri. The cell may be a human induced Pluripotent Stem Cell (hiPSC). For example, the cell may be an hiPSC derived from a patient with a disease or disorder. For example, the cell may be an hiPSC derived from a patient diagnosed or at risk of developing Parkinson's Disease. The cell may be an hiPSC derived from a patient diagnosed with or at risk of developing Dementia with Lewy Bodies.

Methods of introducing a nucleic acid into a host cell are known in the art, and any known method can be used to introduce a nucleic acid (e.g., an expression construct) into a cell. Suitable methods include, include e.g., viral or bacteriophage infection, transfection, conjugation, protoplast fusion, polycation or lipid:nucleic acid conjugates, lipofection, electroporation, nucleofection, immunoliposomes, calcium phosphate precipitation, polyethyleneimine (PEI)-mediated transfection, DEAE-dextran mediated transfection, liposome- mediated transfection, particle gun technology, calcium phosphate precipitation, direct micro injection, nanoparticle-mediated nucleic acid delivery, and the like. In some embodiments, the composition may be introduced by mRNA delivery and ribonucleoprotein (RNP) complex delivery.

Another subject-matter of the invention is a pharmaceutical composition comprising at least one of the composition, the isolated polynucleotide, the vector, or the host cell according to the invention, or combinations thereof.

The disclosure provides for pharmaceutical compositions comprising the composition, isolated polynucleotide, vector, or host cell for modification of the expression of a *SNCA* gene. The pharmaceutical composition may comprise about 1 ng to about 10 mg of DNA encoding the composition, polynucleotide, vector, or host cell for modification of the expression of a *SNCA* gene. For example, about 1 ng to about 100 ng, about 10 ng to about 250 ng, about 50 ng to about 500 ng, about 100 ng to about 750 ng, about 500 ng to about 1 mg, about 750 ng to about 2 mg, about 1 mg to about 5 mg, 2 mg to about 6 mg, about 3 mg to about 7 mg, about 4 mg to about 8 mg, about 5 mg to about 10 mg, or any value in between. The pharmaceutical compositions according to the present invention are formulated according to the mode of administration to be used. In cases where pharmaceutical compositions are injectable pharmaceutical compositions, they are aqueous, sterile-filtered and pyrogen free. An isotonic formulation is preferably used. Generally, additives for isotonicity may include sodium chloride, dextrose, mannitol, sorbitol, lactose, and any combinations of the foregoing. In some cases, isotonic solutions such as phosphate buffered saline are preferred. In some cases, the pharmaceutical compositions further comprise one or more stabilizers. Stabilizers include, but are not limited to, gelatin and albumin. In some embodiments, a vasoconstriction agent is added to the formulation.

The pharmaceutical composition may further comprise a pharmaceutically acceptable excipient. The pharmaceutically acceptable excipient may be functional molecules as vehicles, adjuvants, carriers, or diluents. The method of administration will dictate the type of carrier to be used. Any suitable pharmaceutically acceptable excipient for the desired method of administration may be used. The pharmaceutically acceptable excipient may be a transfection facilitating agent. The transfection facilitating agent may include surface active agents, such as immune-stimulating complexes (ISCOMS), Freund's incomplete adjuvant, LPS analog including monophosphoryl lipid A, muramyl peptides, quinone analogs, vesicles such as squalene and squalene, hyaluronic acid, lipids, liposomes, calcium ions, viral proteins, polyanions, polycations, or nanoparticles, or other known transfection facilitating agents. The transfection facilitating agent may be a polyanion, polycation, including poly-L-glutamate (LGS), or lipid. The transfection facilitating agent may be poly-L-glutamate. The poly-L-glutamate may be present in the pharmaceutical composition at a concentration less than 6 mg/ml. The pharmaceutical composition may include transfection facilitating agent(s) such as lipids, liposomes, including lecithin liposomes or other liposomes known in the art, as a DNA-liposome mixture, calcium ions, viral proteins, polyanions, polycations, or nanoparticles, or other known transfection facilitating agents. Preferably, the transfection facilitating agent is a polyanion, polycation, including poly-L-glutamate (LGS), or lipid.

A still further subject-matter of the invention is a kit comprising at least one of the composition, the isolated polynucleotide, the vector, or the host cell according to the invention, or combinations thereof.

The kit provided herein may be used for modification of the expression of a *SNCA* gene. A "kit" is a combination of individual elements useful for carrying out the invention, wherein the elements are optimized for use together in the methods. The kits may also contain additional reagents, chemicals, buffers, reaction vials etc. which may be useful for carrying out the invention. The kit may comprise instructions for using the disclosed composition, polynucleotide, vector, or pharmaceutical composition for modification of a *SNCA* gene expression. Instructions included in kits may be affixed to packaging material or may be included as a package insert. While the instructions are typically written or printed materials, they are not limited to such. Any medium capable of storing such instructions and communicating them to an end user is contemplated by this disclosure. Such media include, but are not limited to, electronic storage media (e.g., magnetic discs, tapes, cartridges, chips), optical media (e.g., CD ROM), and the like. As used herein, the term "instructions" may include the address of an internet site that provides the instructions. Such kits unify all essential elements required to work the invention, thus minimizing the risk of errors. Therefore, such kits also allow semi-skilled laboratory staff to perform the invention.

Another subject-matter of the invention relates to a method of modulation of expression of the *SNCA* gene in a biological cell or a subject, the method comprising contacting the cell or subject with at least one of the composition, the isolated polynucleotide, the vector, the host cell, or the pharmaceutical composition according to the invention, or combinations thereof, in an amount sufficient to modulate expression of the *SNCA* gene.

The present disclosure provides for methods of *in vivo* and *in vitro* modulation of expression of a *SNCA* gene. The method can include modulation of expression of a *SNCA* gene in a biological cell. The method can include modulation of expression of a *SNCA* gene in a subject. The method can include administering to the cell or subject the presently disclosed composition, polynucleotide, vector, host cell, or pharmaceutical composition for modification of the expression of a SNCA gene. The method can include administering to the cell or subject a pharmaceutical composition comprising the same.

The invention also relates to a method of treating a disease or disorder associated with altered, preferably elevated *SNCA* gene expression levels in a subject, the method comprising administering to the subject or a biological cell in the subject at least one of the composition, the isolated polynucleotide, the vector, the host cell, or the pharmaceutical composition according to the invention, or combinations thereof, in an amount sufficient to modulate expression of the *SNCA* gene.

The disclosure provided above for the "host cell" according to the invention applies to the cell recited in the method according to the invention in the same manner.

"Subject" and "patient" as used herein interchangeably refers to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous or rhesus monkey, chimpanzee, etc.) and a human). In some embodiments, the subject may be a human or a non-human. The subject or patient may be undergoing other forms of treatment.

The features, characteristics, advantages and embodiments specified herein with respect to the composition according to the invention apply to the isolated polynucleotide, the vector, the host cell, the pharmaceutical composition, the kit, and the methods according to the invention in a corresponding manner, even if not specifically indicated.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

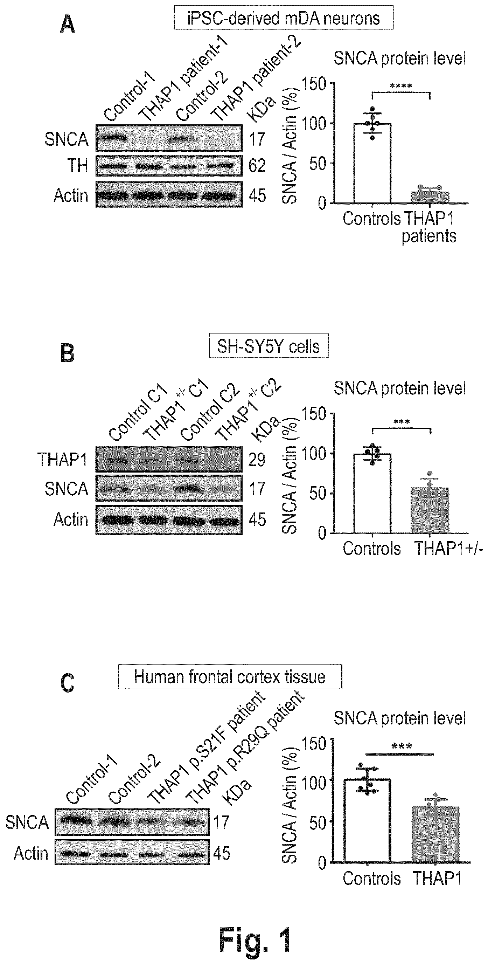

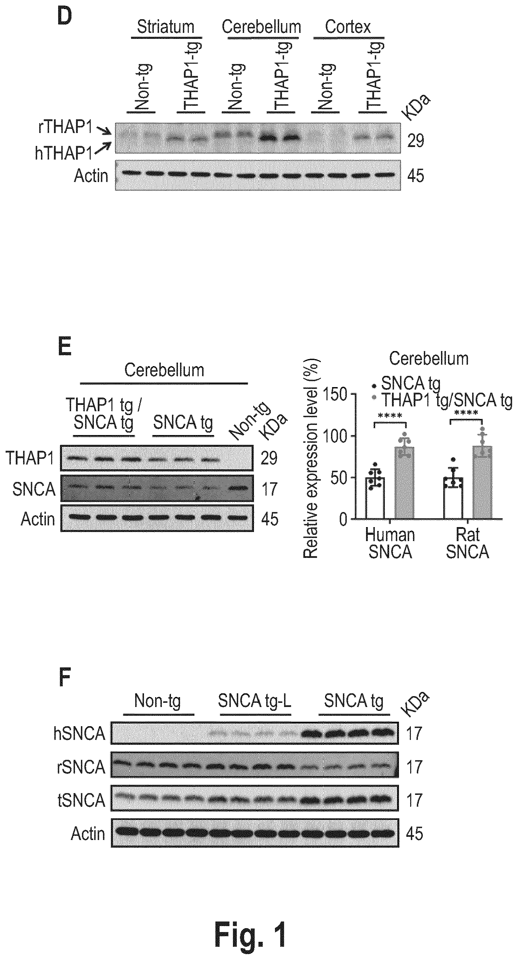

- Fig. 1:: THAP1 regulates the expression of SNCA. (A) Western blotting and statistical analysis show that the expression of SNCA in THAP1 patients' iPSC-derived mDA neurons is significantly lower than in control iPSC-derived mDA neurons. (B) In THAP1 heterozygous knock-out (THAP1^{+/-}) dopaminergic SH-SY5Y cells western blotting and statistical analysis detect that the expression of THAP1 and SNCA are significantly lower than in control cells. (C) The expression of SNCA in THAP1 patients' frontal cortex is lower than in control individuals by Western blotting and statistical analysis. (D) Western blot analysis shows the expression of human THAP1 (hTHAP1) and rat THAP1 (rTHAP1) proteins in different brain regions of THAP1 transgenic rats (n=3 rats in each group). (E) Western blotting and statistical analysis determine the protein level of human SNCA (hSNCA) and rat SNCA (rSNCA) in the cerebellum of human THAP1 and SNCA double transgenic rats (THAP1 tg/ SNCA tg), SNCA transgenic rats (SNCA tg), and non-transgenic wild-type rats (Non-tg) (n=5 rats in each group). (F) Western blotting shows the protein level of hSNCA, rSNCA, and total SNCA (tSNCA) in Non-tg and two different SNCA overexpressing transgenic rat lines (SNCA tg-L and SNCA tg) (n=5 rats in each group). (***: p<0.001; ****: p<0.0001. Comparison by unpaired two-tailed t test)

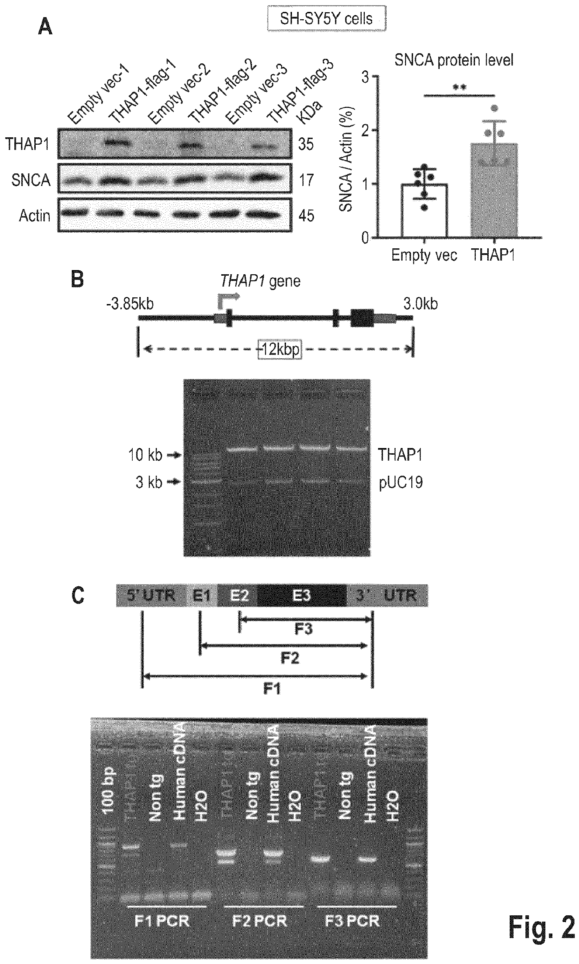

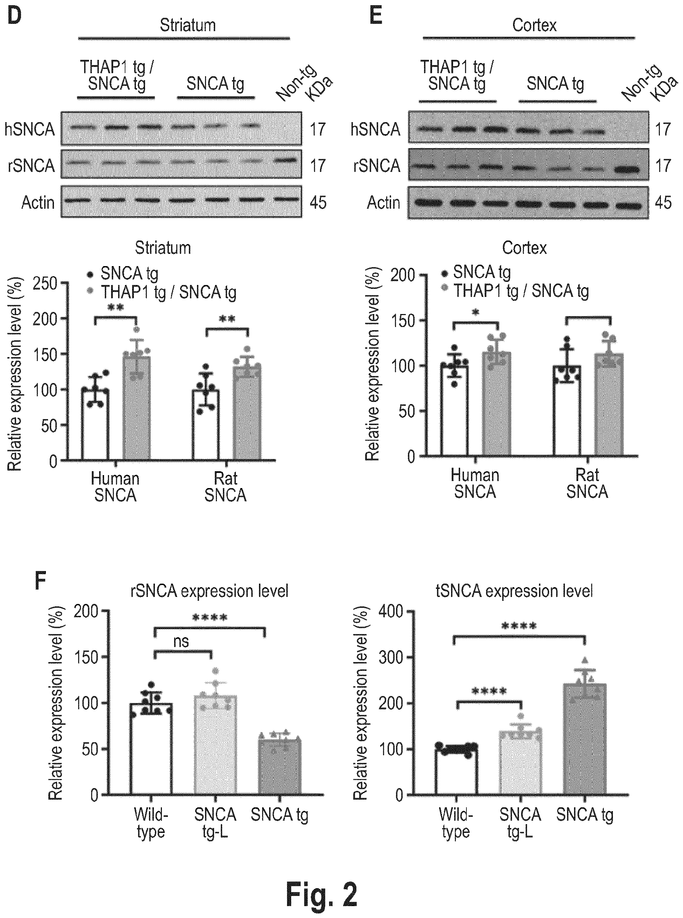

- Fig. 2:: (A) Western blotting and statistical analysis show the SNCA protein level in THAP1 overexpression cell lines (THAP1-flag). (B) Strategy to clone the whole human *THAP1* gene (12 kb), including upstream (-3.85 kb) and downstream (3.0 kb) regions of this gene, into pUC19 vector (upper). Restriction enzyme digestion and electrophoresis analysis validate the results of positive clones (bottom). (C) Reverse-transcript PCR analyzes the expression of THAP1 mRNA in THAP1 tg rats. Fragment 1 (F1) and fragment 2 (F2) RT-PCR products both have two bands, because *THAP1* gene has an exon 2 skipping transcripts. (D and E) Western blotting (top) and statistical analysis (bottom) determine the protein level of human SNCA (hSNCA) and rat SNCA (rSNCA) in Striatum (D) and Cortex (E) of human THAP1 and human SNCA double transgenic rats (THAP1 tg/ SNCA tg), SNCA transgenic rats (SNCA tg), and non-transgenic wild-type rats (Non-tg). (F) Statistical analysis of rSNCA and total SNCA (tSNCA) in wild-type, SNCA tg-L, and SNCA tg rats. (*: p<0.05; **: p<0.01; ****: p<0.0001. Comparison by unpaired two-tailed *t* test).

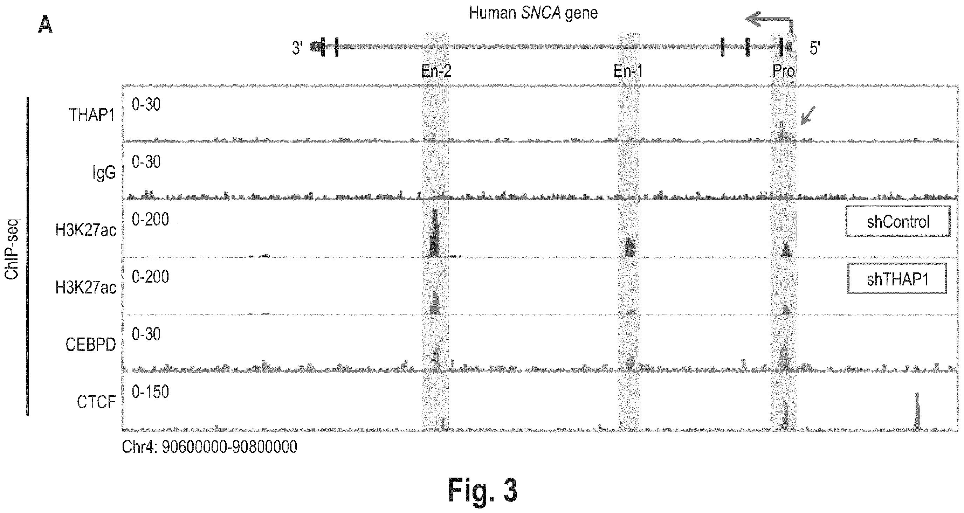

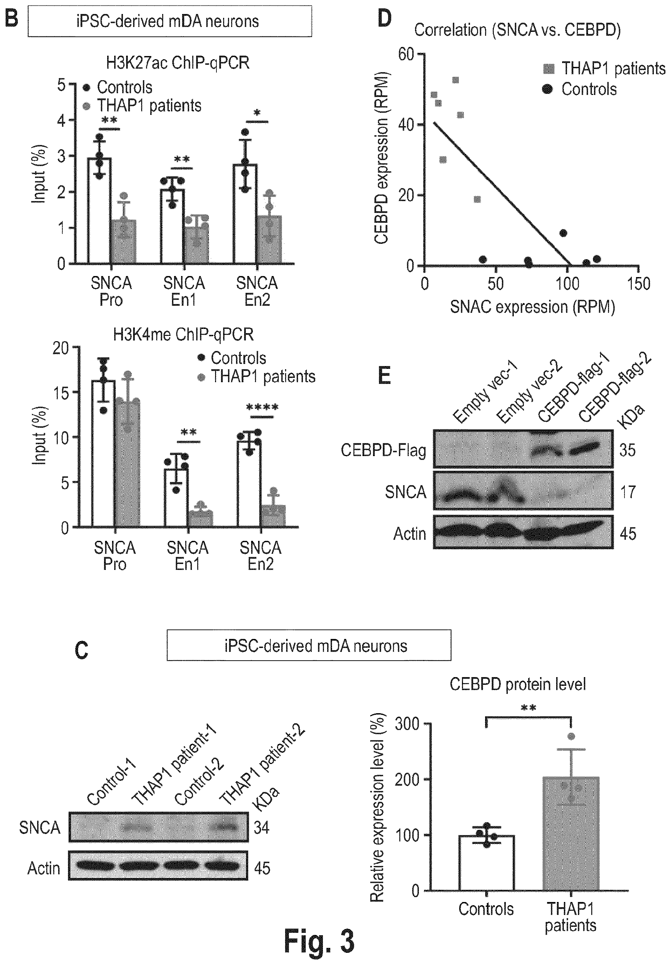

- Fig. 3:: Characterization the mechanisms by which THAP1 regulate SNCA expression. (A) THAP1 ChlP-seq detects its binding signal on SNCA promoter (red arrow) which is not seen in IgG control (lane 1 and 2). H3K27ac ChlP-seq defines the SNCA promoter and potential enhancers in SH-SY5Y cells and also shows its modification on *SNCA* gene in control (shControl) and THAP1 knock-down (shTHAP1) SH-SY5Y cells (En-1: Chr4: 90720577-90722136; En-2: Chr4: 90673800-90675200) (lane 3 and 4). CEBPD and CTCF ChlP-seq show their binding sites on both SNCA promoter and enhancers (lane 5 and 6). (B) H3K27ac and H3K4me3 ChIP-qPCR analyze their modifications on *SNCA* promoter and enhancers in THAP1 patients' iPSC-derived mDA neurons and in control iPSC-derived mDA neurons. (C) Western blot analysis shows that the endogenous CEBPD protein level in THAP1 patients' iPSC-derived mDA neurons is higher than in control iPSC-derived mDA neurons. (D) Correlation analysis reveal that the CEPBD mRNA level is negatively correlated to the SNCA mRNA level in iPSC-derived mDA neurons (RPM: reads per million) (Equation: Y= -0.4196 * X + 43.28; *p*=0.0014). (E) Western blotting determines the expression of CEBPD and SNCA in CEBPD overexpressing SH-SY5Y cells. (*: p<0.05; **: p<0.01; ****: p<0.0001. Comparison by unpaired two-tailed *t* test)

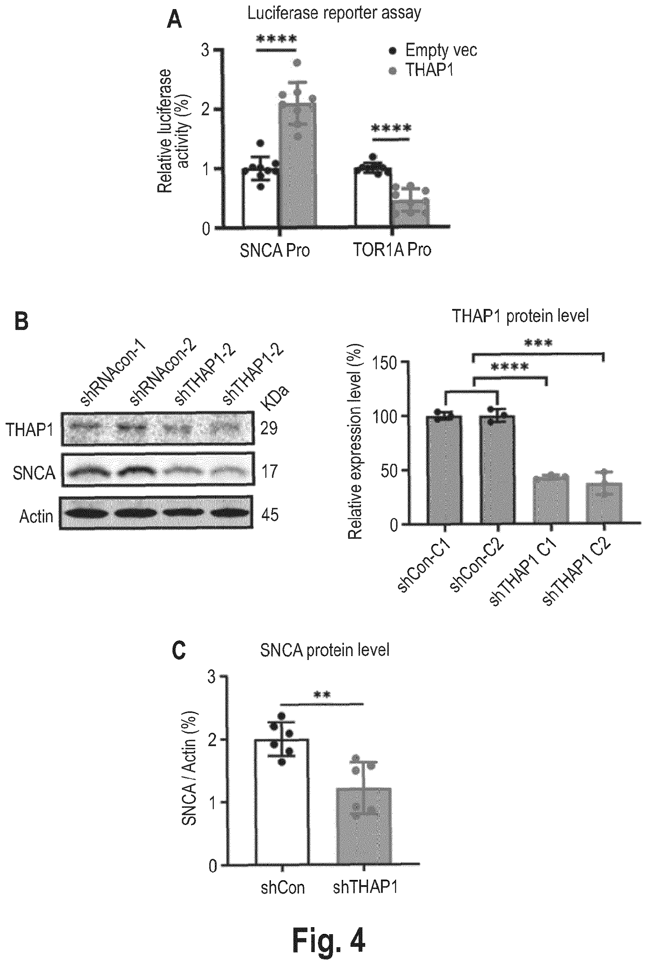

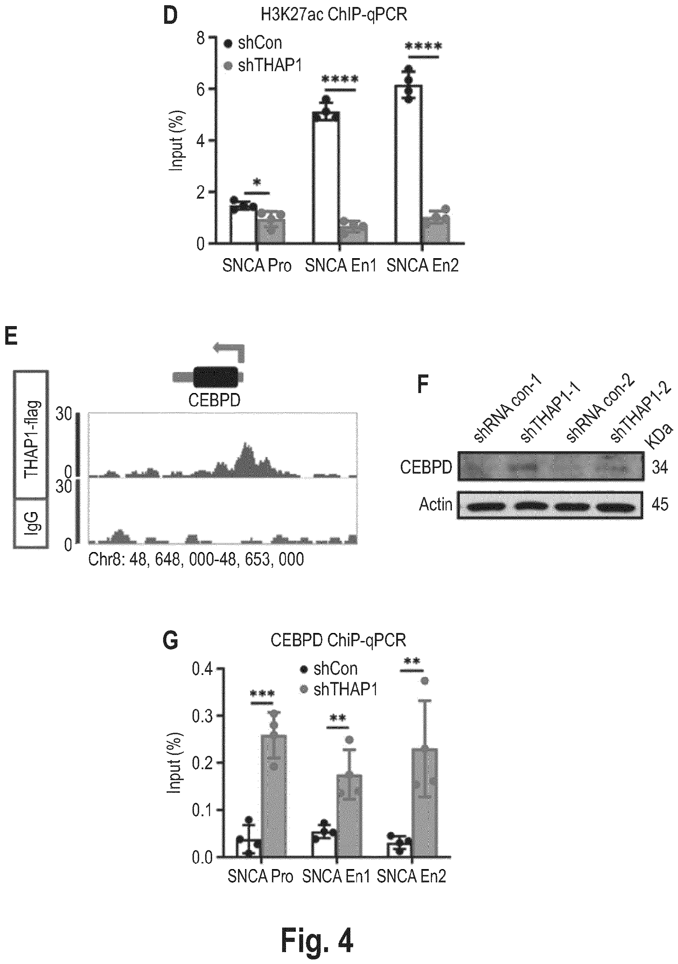

- Fig. 4:: (A) Luciferase reporter assays analyze the role of THAP1 in regulating SNCA promoter and TOR1A promoter activities. The SNCA promoter (SNCA pro, 10.7 kb) or TOR1A promoter (TOR1A pro) were co-transfected with wild-type THAP1 into SH-SY5Y cells, respectively, to determine their interaction. TOR1A pro was used as positive control. (B) Western blotting and statistical analysis determine the protein level of THAP1 and SNCA in THAP1 knock-down cell lines (shTHAP1) and control cell lines (shCon). (C) Statistical analysis of the SNCA protein level in shTHAP cell lines compared to shCon cell lines. (D) H3K27ac ChIP-qPCR analyze the H3K27ac modification on SNCA promoter (SNCA pro) and enhancers (SNCA En1: SNCA enhancer 1 region; SNCA En2: SNCA enhancer 2 region) in shTHAP1 cell lines and shCon cell lines. (E) Anti-flag ChIP-seq data (using anti-flag antibody and THAP1-flag overexpression cell lines) detect the THAP1 binding site on *CEBPD* gene. (F) Western blotting analyzes the CEBPD protein level in shTHAP1 cells and shCon cells (left). (G) CEPBD ChIP-qPCR shows its binding activities on SNCA promoter (SNCA pro) and enhancers (SNCA En1 and SNCA En2) in shTHAP1 cell lines and shCon cell lines (right). (*: p<0.05; **: p<0.01; ***: p<0.001; ****: p<0.0001. Comparison by unpaired two-tailed t test).

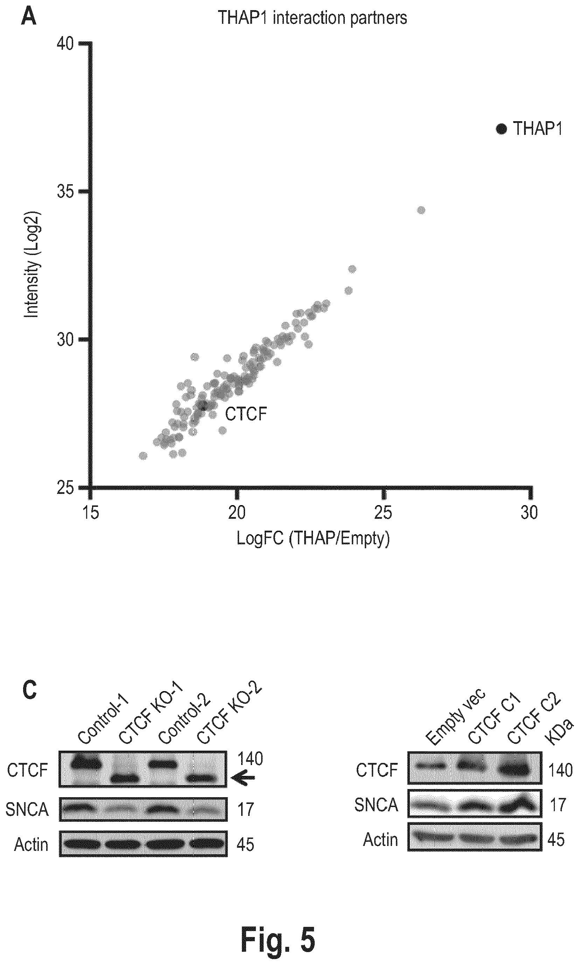

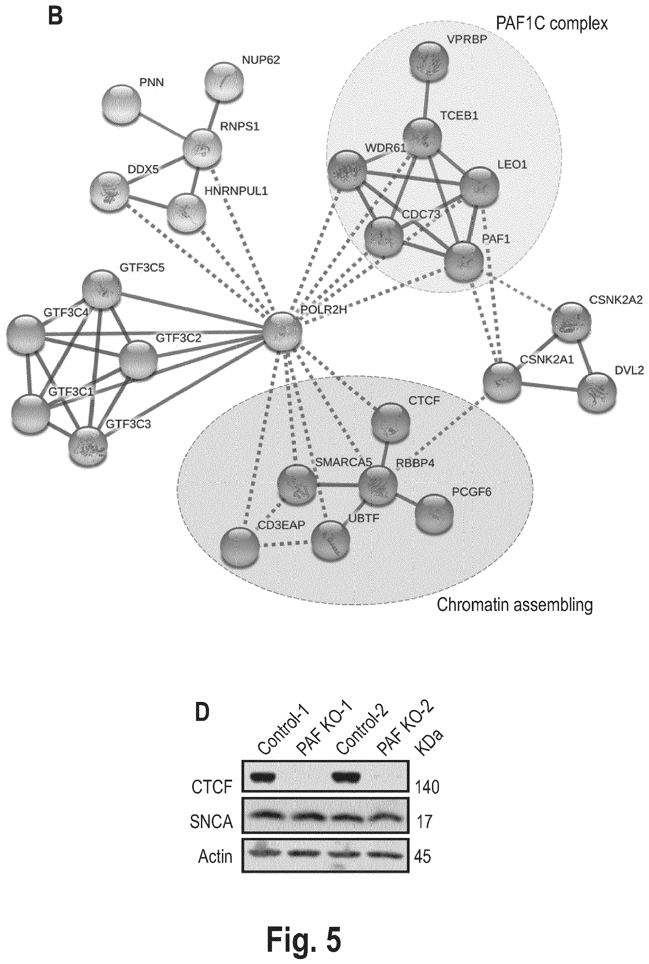

- Fig. 5:: THAP1 interaction partner CTCF regulates the expression of SNCA. (A) Dot plots presents the interaction partners of THAP1 identified by TAP-MS analysis, CTCF and THAP1 are marked in red color. (B) String-db network analysis anno-tated the protein complex of THAP1 interaction partners. (C) Western blot analyses detect the protein level of CTCF and SNCA in CTCF knock-out (left) and CTCF overexpressing (right) SH-SY5Y cells. (Black arrow indicates a small isoform of CTCF 1). (D) Western blotting analyzes the protein level of PAF1 and SNCA in PAF1 knock-out SH-SY5Y cells.

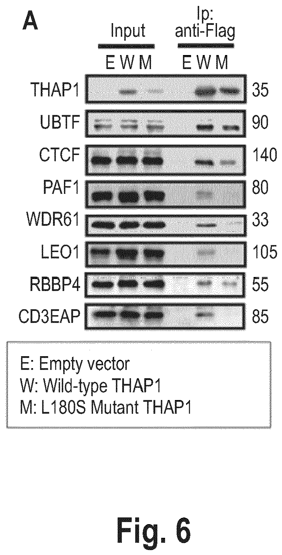

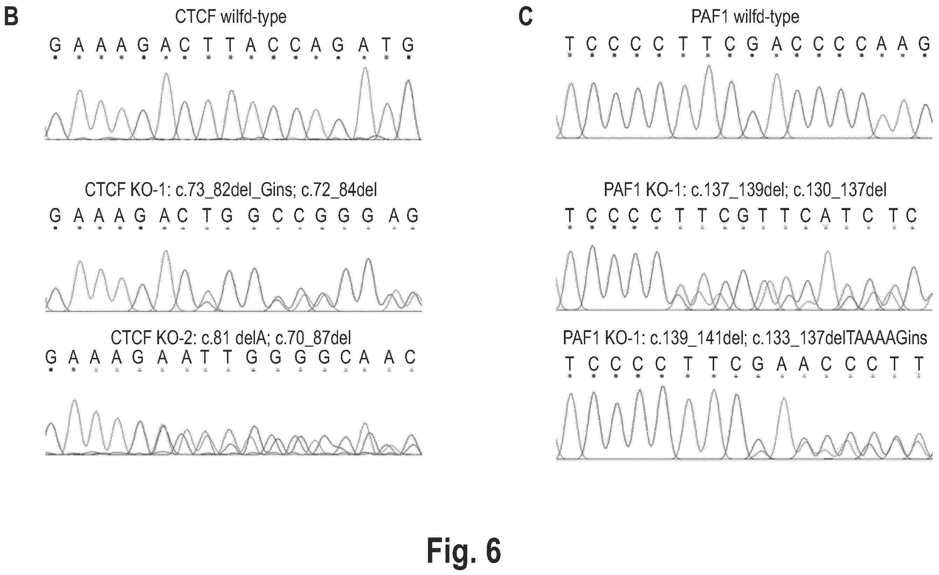

- Fig. 6:: (A) Western blot analysis to determine the interactions between THAP1 and some of its potential interaction partners isolated by Tandem Affinity Purification and Mass Spectrometry analysis (TAP-MS). (B) Sanger sequencing of genotype results of two CTCF knock-out SH-SY5Y clones (CTCF KO-1 and CTCF KO-2). (C) Sanger sequencing of genotype results of two PAF1 knock-out SH-SY5Y clones (PAF1 KO-1 and PAF1 KO-2).

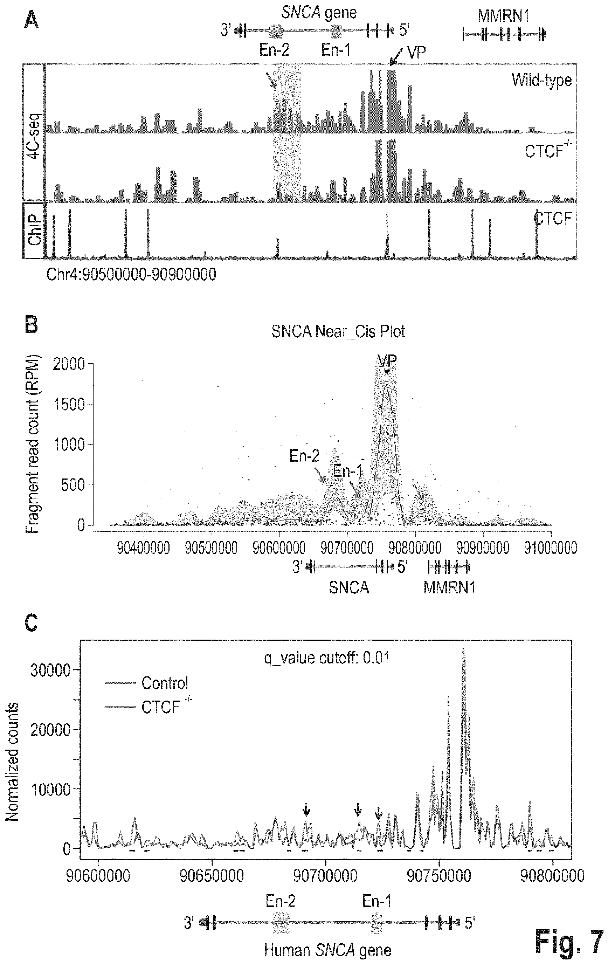

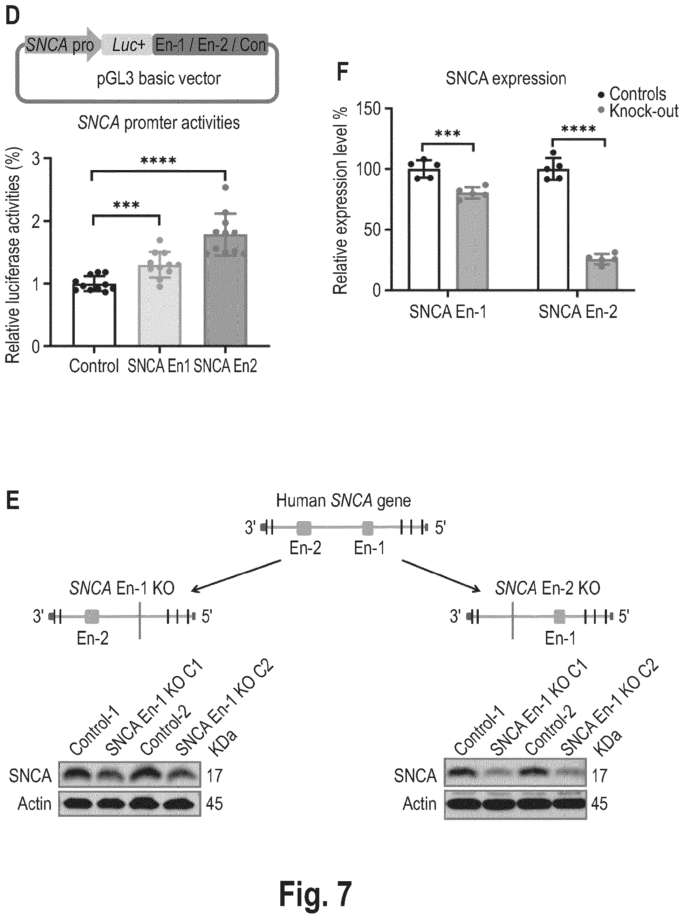

- Fig. 7:: SNCA intronic enhancers regulate its expression. (A) Distribution of SNCA 4C-seq raw reads in wild-type and CTCF^{-/-} SH-SY5Y cells (lane 1 and 2). CTCF ChlP-seq detects its binding sites around SNCA gene (lane 3). SNCA promoter region was set as view point (VP). Red arrow indicates the *SNCA* En-2 region which is strongly contact with the VP in wild-type cells, while this interaction was disappeared in CTCF^{-/-} SH-SY5Y cells (En-1 and En-2 indicate the position of the two *SNCA* intronic enhancers, respectively). (B) Basic4C package was used to analyze the SNCA 4C-seq data. Red arrows indicate the regions that contact with the VP (*SNCA* promoter) (from left to right *SNCA* En-2, *SNCA* En-1, and *MMRN1* promoter). (C) 4C-ker package analyze the change of interactions between *SNCA* promoter and its intron 4 region (including its two enhancers) in wild-type and CTCF knock-out (CTCF^{-/-}) SH-SY5Y cells (at least two biological replicates of each sample were performed). (Black arrows and short black lines both show the regions with statistically changed interactions with the *SNCA* promoter) (4C-ker analysis default setting: K=10, q_value =0.01). (D) Schematic diagram shows the strategies of cloning vectors to characterize the role of *SNCA* enhancers (En-1 and En-2) in regulating its promoter (*SNCA* pro) activities. Genomic unrelated region was used as negative control (Con) (top). Luciferase reporter assay analysis determines the role of *SNCA* enhancers in regulating its promoter activities (bottom). (E) Schematic graphs present the strategies using CRISPR/Cas9 technique to knock-out *SCNA* En-1 or En-2 in SH-SY5Y cells (top). Western blotting (bottom) shows the SNCA protein level in En-1 or En-2 knock-out SH-SY5Y cells. (F) Statistical analysis shows the SNCA protein level in *SNCA* En-1 or En-2 knock-out SH-SY5Y cells. (***: p<0.001; ****: p<0.0001. Comparison by unpaired two-tailed *t* test).

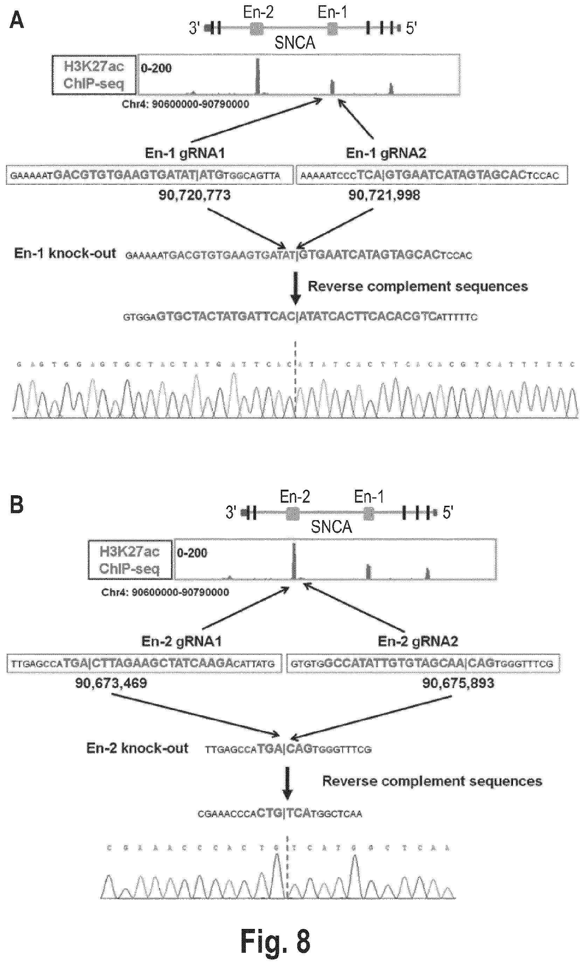

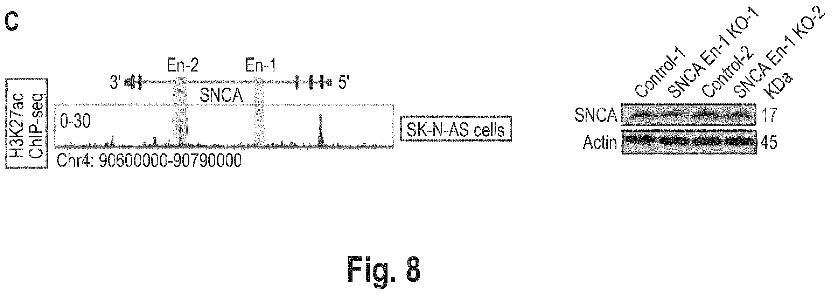

- Fig. 8:: (A and B) The strategies and gRNA sequences used to knock-out of *SNCA* En-1 (A) and En-2 (B) in SH-SY5Y cells (top). Sanger-sequencing determines the positive knock-out clones (bottom). (C) H3K27ac ChlP-seq detected the *SNCA* enhancer and promoter in SK-N-AS neuroblastoma cells. The *SNCA* En-1 region is inactivated in this cell line (Left). Western blotting shows the SNCA protein level in control and *SNCA* En-1 KO SK-N-AS cells (Right).

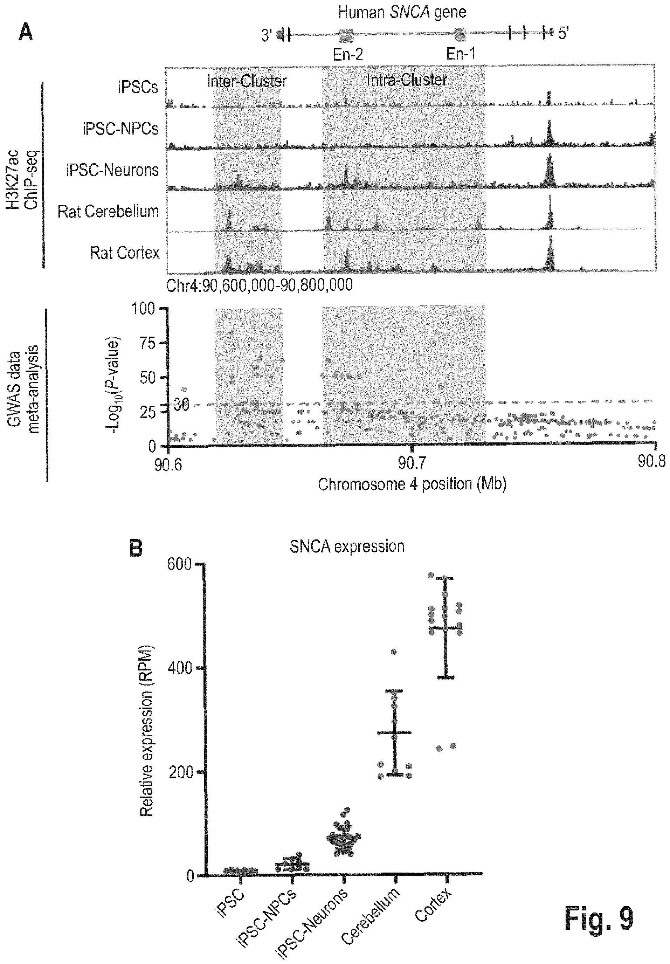

- Fig. 9:: Lineage-dependent activation of *SNCA* enhancers are associated to its expression and PD risk. (A) The status of H3K27ac modification of the human *SNCA* gene in iPSCs, iPSC derived neural progenitor cells (iPSC-NPC), iPSCs derived mDA neurons (iPSC-Neurons), frontal cortex of SNCA tg rats (Rat Cortex), and cerebellum of SNCA tg rats (Rat Cerebellum) (top). Dot plots show the distribution of PD risk associated SNPs on *SNCA* gene by meta-analysis of GWAS data (*Nalls et al., 2014*). Orange dots indicate SNPs with extremely higher association with PD risk (-Log₁₀(*P*-values) > 30) (bottom). Inter-Cluster and Intra-Cluster indicate the enhancer clusters down-stream of the *SNCA* gene and the intronic enhancer clusters, respectively. (B) Relative expression level of SNCA in different cells and tissues determined by RNA-seq analysis (RPM: reads per million).

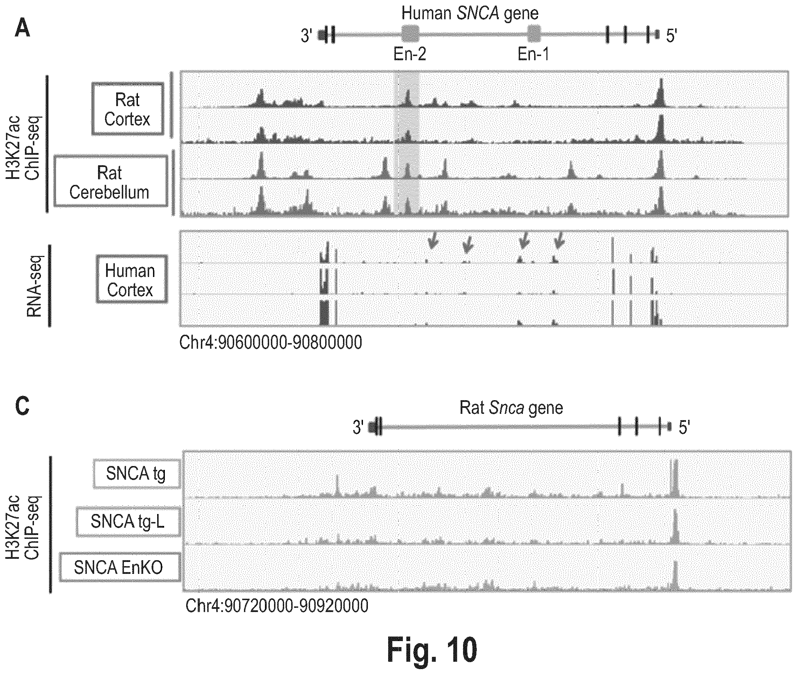

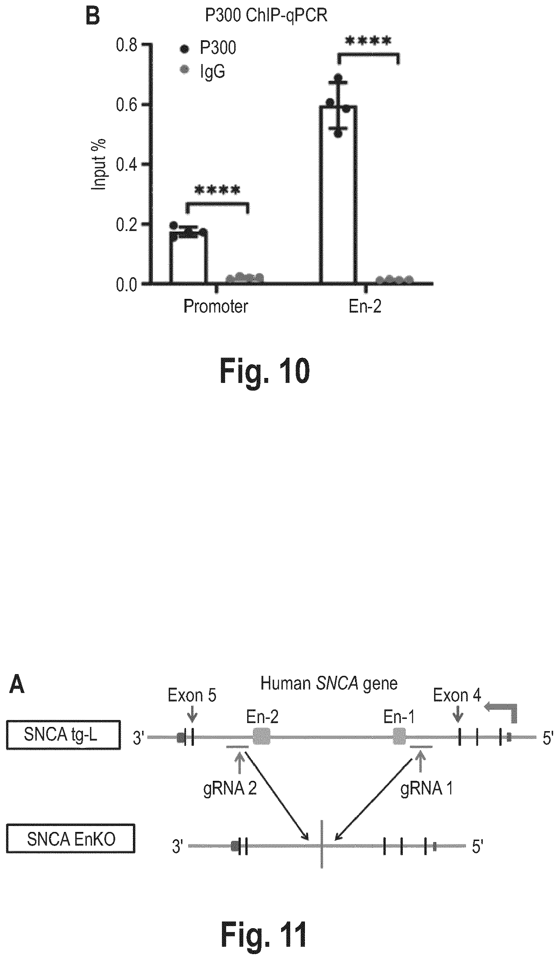

- Fig. 10:: (A) The H3K27ac modification status of human *SNCA* gene in cortex and cerebellum of SNCA tg rats and SNCA tg-L rats (top). RNA-seq analysis of total RNA isolated from human frontal cortex (bottom) (red arrows indicate the expression of enhancer RNA, eRNA). Grey squares indicate the enhancer region of *SNCA* En-2.(B) ChIP-qPCR analysis of the P300 binding activities on *SNCA* promoter and En-2 in SNCA tg rat cortex. (C) The status of H3K27ac modification on rat *Snca* gene in cortex of SNCA tg rats, SNCA tg-L, and SNCA EnKO rats (****: p<0.0001. Comparison by unpaired two-tailed t test).

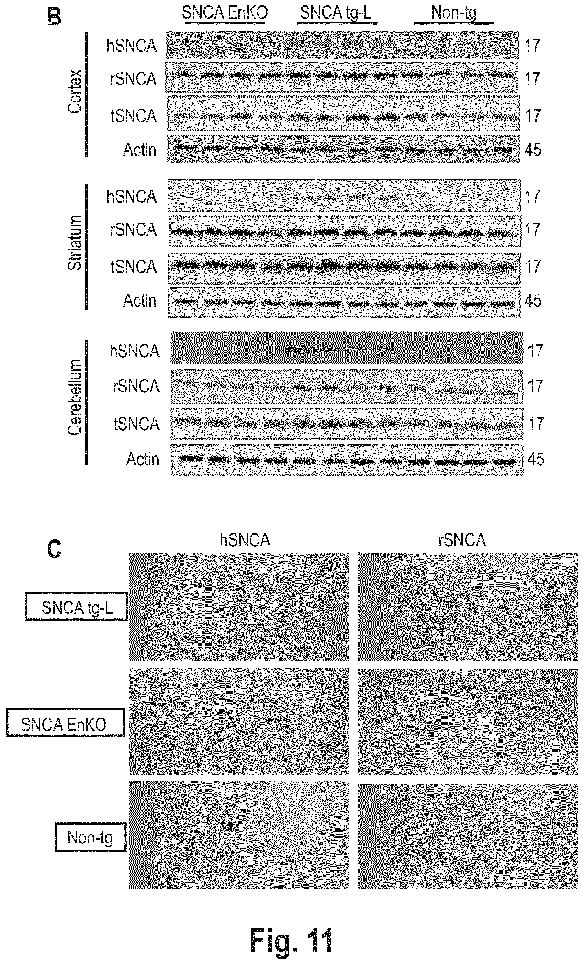

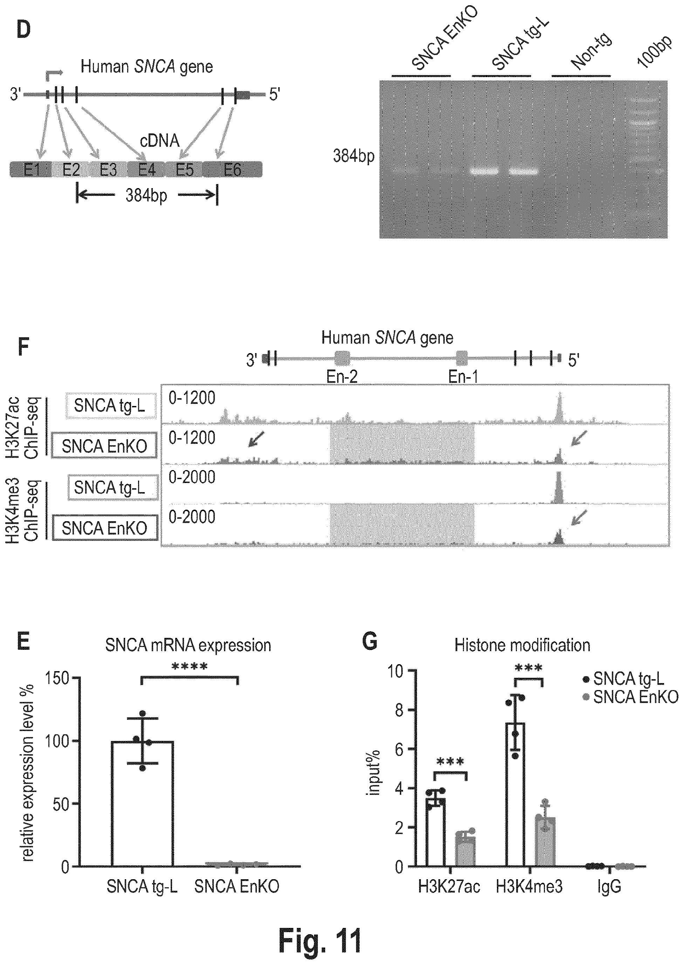

- Fig. 11:: The human *SNCA* intronic enhancer clusters predominantly control its expression in brain. (A) Schematic graph show the position of gRNAs and the human *SNCA* gene structure in SNCA tg-L and SNCA EnKO rats. (B) Western blotting confirms the expression of hSNCA, rSNCA, and tSNCA in different brain regions of SNCA EnKO, SNCA tg-L, and Non-tg rats (n=5 rats in each group). (C) Immunohistochemistry staining of hSNCA and rSNCA in the brain of SNCA tg-L, SNCA EnKO, and Non-tg rats (n=3 rats in each group). (D) Schematic representation of the primers used to amplify full-length human SNCA mRNA (384bp in length) (left). The reverse-transcript PCR determines the expression of full length human *SNCA* mRNA in SNCA EnKO, SNCA tg-L, and Non-tg rats (right) (n=3 rats in each group). (E) Reverse-transcript quantitative PCR (RT-qPCR) determines the expression level of human *SNCA* mRNA in SNCA EnKO rats. SNCA tg-L rats were used as control (n=5 rats in each group). (F) H3K27ac and H3K4me3 ChIP-seq reveal the status of histone modifications of the human *SNCA* gene in SNCA tg-L and SNCA EnKO rats (blue arrow: H3K27ac modification on down-stream of human *SNCA* gene in SNCA EnKO rat; red arrow: H3K27ac and H3K4me3 modification on promoter of human *SNCA* gene in SNCA EnKO rat; gray squares indicate the region that was cut from the human *SNCA* gene in SNCA EnKO rat). (G) ChIP-qPCR quantitatively determines the histone modifications on the SNCA promoter in SNCA tg-L and SNCA EnKO rats (n=4 rats in each group). (***: p<0.001; ****: p<0.0001. Comparison by unpaired two-tailed *t* test).

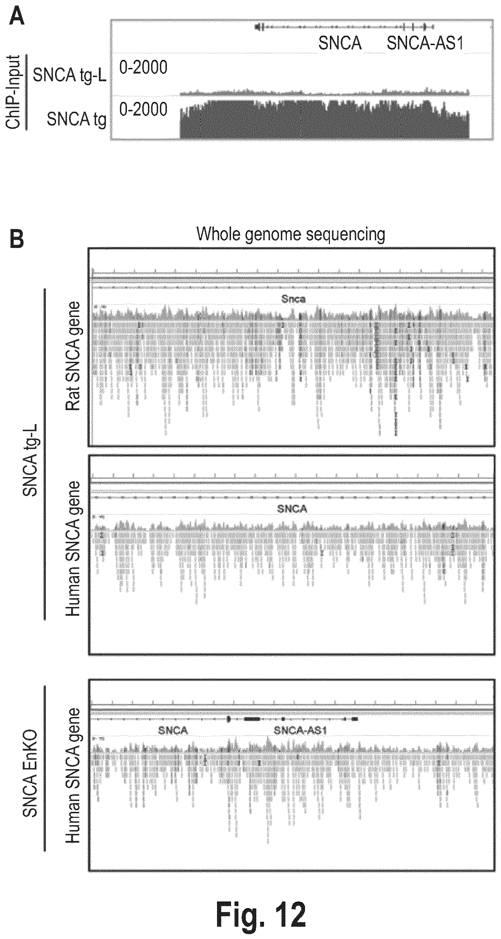

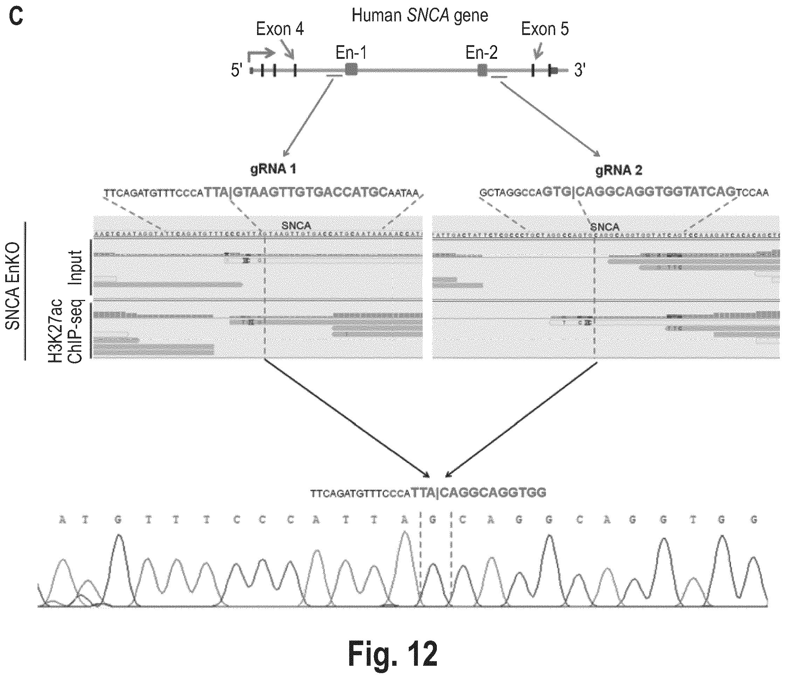

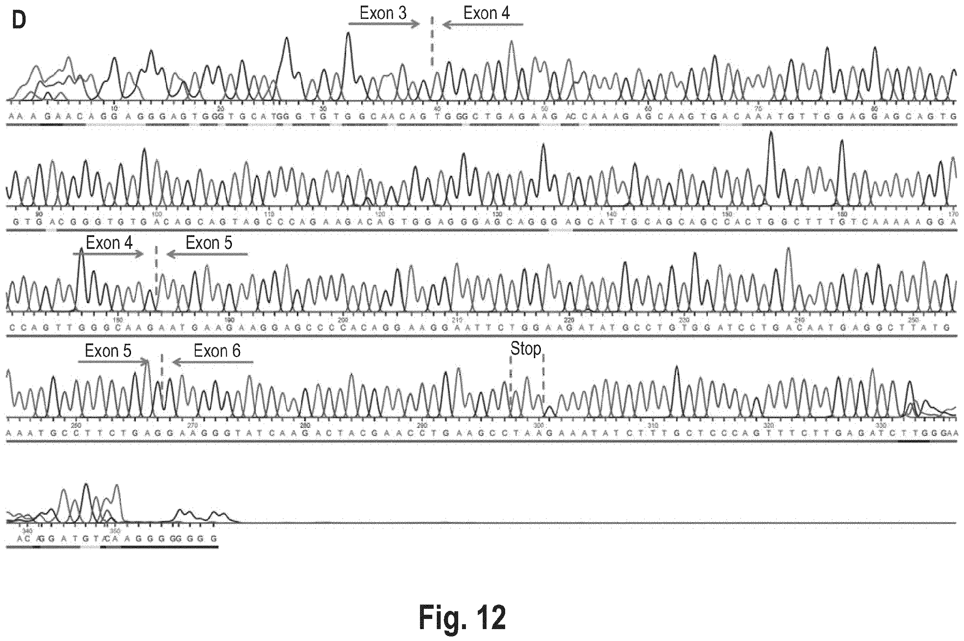

- Fig. 12:: (A) IGV gene browser view the whole genome sequencing raw reads intensity of human *SNCA* gene in SNCA tg-L rats and SNCA tg rats. (B) IGV Gene browser overview of reads intensity of rat *Snca* gene and human *SNCA* gene in SNCA tg-L rats (top). IGV gene browser view of reads intensity of human *SNCA* gene in SNCA EnKO rats (bottom). (C) Schematic graph presents the location of gRNA and *SNCA* gene structure (top). IGV gene browser view the cutting sites generated by CRISPR/Cas9 techniques (middle). Sanger-sequencing confirmed the re-combination of two residual ends after knocking-out the *SNCA* large intronic enhancer. (D) Sanger-sequencing of hSNCA cDNA PCR product amplified from SNCA EnKO cDNA. No aberrant splicing or exon skipping was found.

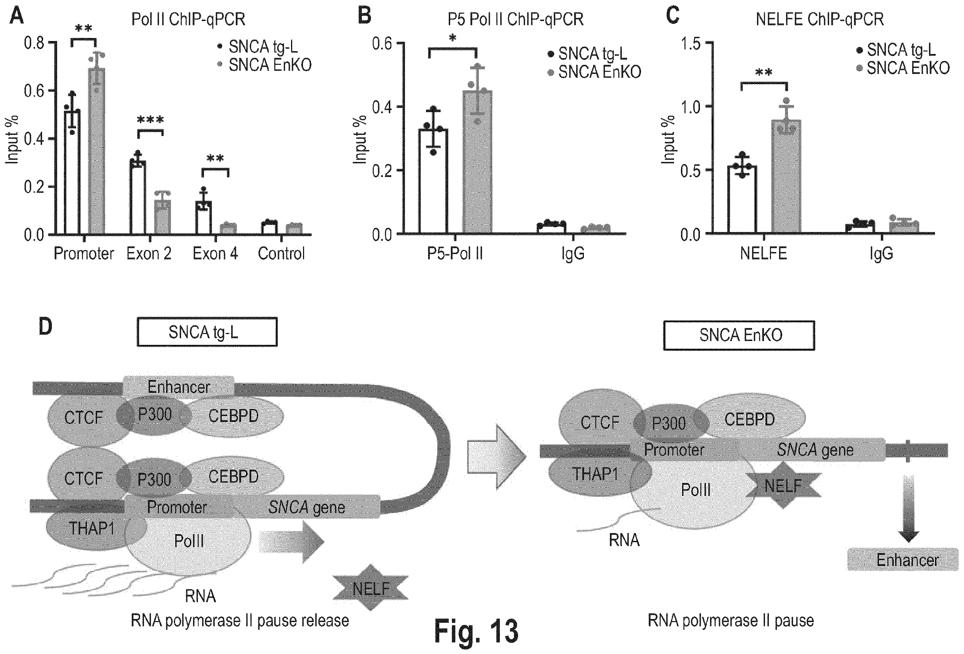

- Fig. 13:: The human *SNCA* intronic enhancer clusters promote releasing of paused RNA polymerase II. (A) ChIP-qPCR quantitative analyze the binding activities of polymerase II (Pol II) on the human *SNCA* promoter and different regions of gene body (exon 2 and exon 4) in SNCA tg-L and SNCA EnKO rat cortex. (Control: unrelated genomic region 10 kb upstream of the *SNCA* gene) (n=4 rats in each group). (B) ChIP-qPCR quantitative analysis shows the binding of phosphorylated (Ser5) polymerase II (P5 Pol II) on the human *SNCA* promoter in SNCA tg-L and SNCA EnKO rat cortex (n=4 rats in each group). (C) ChIP-qPCR analysis determines the binding activities of negative elongation factor E (NELFE) to the human *SNCA* promoter in SNCA tg-L and SNCA EnKO rat cortex (n=4 rats in each group). (D) Schematic graph show the structures and transcription factors of the human *SNCA* gene in wild-type (*SNCA* tg-L) and knock-out enhancer clusters (SNCA EnKO) conditions. The human *SNCA* promoter was bound by many transcription factors and contacts with its intronic enhancer (Enhancer). The contact between promoter and enhancer promotes releasing of polymerase II pause and transcription elongation (left). Knocking-out of the intronic enhancer clusters (enhancer) of the human *SNCA* gene loses the contact between enhancer and promoter and increases the enrichment of negative elongation factor (NELF) on its promoter, which cause RNA polymerase II pausing (right). (*: p<0.05; **: p<0.01; ***: p<0.001. Comparison by unpaired two-tailed *t* test).

### EXAMPLES

### 1. Material and methods

### 1.1 Cell culture

All the cell lines were authenticated by using polymorphic short tandem repeat (STR) loci before used for experiment. Mycoplasma contamination was tested regularly every three months. HEK-293 (ATCC^{®} CRL-1573^{™}) and SH-SY5Y (ATCC^{®} CRL-2266^{™}) cells were purchased from Sigma (85120602 and 94030304) and cultured in DMEM medium with 10% FBS and 1% Glutmax at 37 °C in a humidified atmosphere containing 5% CO₂.

### 1.2 Generation of shTHAP1 SH-SY5Y cell lines

Stably knock-down of THAP1 in SH-SY5Y cells was performed by stably transfection of THAP1 shRNA expresson vector (SureSilencing shRNA plasmids KH07515N, QIAGEN). Four shRNA expression vectors were provided and two of them were pre-selected for stably knock-down THAP1 in SH-SY5Y cells (Target 1: GACGTATCAGAAAGAGGTTAT (SEQ ID NO: 9); Target 2: GCCTGTAAAGGAGTTTCATTT (SEQ ID NO: 10)) because these two shRNA can sufficiently repress the endogenous *THAP1* mRNA level after transient transfection in cells (data not show). The expression of THAP1 was determined by both real-time PCR and Western blot. Control cell lines were stably transfection of negative control shRNA (Sequences: GGAATCTCATTCGATGCATAC (SEQ ID NO: 11)).

### 1.3 Plasmid vectors

Human THAP1 cDNA ORF was amplified by PCR from cDNA of HEK-293 cells and cloned into pcDNA3.1 or pcDNA 3.1/Flag plasmid (Invitrogen). CEBPD expression vector was purchased from Genscript (OHu17203D; Genscript). pX330-U6-Chimeric_BB-CBh-hSpCas9 was a gift from Feng Zhang (Addgene plasmid # 42230; http://n2t.net/addgene:42230). pSpCas9(BB)-2A-Puro (PX459) V2.0 was a gift (Addgene plasmid # 62988). pBABE-puro was a gift (Addgene plasmid # 1764; http://n2t.net/addgene:1764). CTCF expression vector were generated by amplifying its cDNA from pDONR223_CTCF_WT (Addgene plasmid #81789) and subcloning into pcDNA3.1/V5tag vector. All subcloned vectors were verified by Sanger sequencing before used.

### 1.4 Generation of induced pluripotent stem cells (iPSCs) and differentiation into midbrain dopaminergic neurons (mDA)

Generation of iPSCs and differentiation into mDA neuonrs have been described in the art. The ethical approval for the development of and research pertaining to patient-derived cell lines has been given by the National Committee for Ethics in Research (CNER, Luxembourg). In brief, native fibroblasts were obtained from skin biopsies with the written informed consent of donors. Fibroblasts were maintained and cultured as previously described in the art. The information of patients and control individuals were published previously in the art. Fibroblasts were reprogrammed to pluripotency using the Simplicon^{™} RNA reprogramming kit (Merck). Molecular karyotyping and identity analysis was performed using HumanOmni 2.5 Exome-8 DNA Analysis BeadChip (Life&Brain GmbH). The iPS cells were cultured as previously published in the art.

Midbrain dopaminergic (mDA) neurons were differentiated from iPSCs according to the previously reported protocols. iPSCs were first derived into neural precursor cells (NPCs) using N2B27 media as described in the art. The NPCs were directly differentiated in N2B27 media containing 1 µM Purmorphamine, 200 µM Ascorbic Acid, and 100 ng/mL FGF8b (Peprotech, 100-25). After 8 days of differentiation, the culture medium was change to N2B27 supplemented with 0.5 µM Purmorphamine and 200µM Ascorbic Acid for another two days. From 10 days of directed differentiation until the designated experimental timepoint (d45 onwards) the following substances were added to N2B27 media, BDNF (10 ng/MI; Peprotech, 450-02), GDNF (10 ng/mL; Peprotech, 450-10), TGFβ3 (1 ng/mL; Peprotech, 100-21), cAMP (500µM; Applichem, A0455) and Ascorbic Acid (200 µM) .

### 1.5 Human post-mortem brain tissues

Frozen post-mortem frontal cortex tissue was obtained from two THAP1 patients (with THAP1 p.S21F and p.R29Q mutation, respectively) and three neurological normal control individuals as described in the art.

### 1.6 CRISPR/Cas9 knock-out CTCF and PAF1

Knock-out of CTCF and PAF1 were performed using CRISPR/Cas9 technique. Guide RNA were selected by CRISpick (https://portals.broadinstitute.org/gppx/crispick/public). Guide RNAs oligos synthesized by biomers (www.biomers.net) were annealed and sub-cloned into pX459 vector. Transfection was done by co-transfect of pX459/gRNA and pBabe empty vector (ratio 10:1) into SH-SY5Y cells. Twenty-four hours after transfection, cells were selected by puromycin (final concentration 2ug/ ml) for another 48 hours. The surviving cells were sub-cultured in 10 cm dishes until single clone formation. Sanger sequencing and western blot were used to select positive cell clones and verify target protein expression. All gRNA sequence and genotype primers were list in table 1.

**Table 1: List of gRNA sequences and genotype PCR primers.**

| Targets | gRNA sequences | Positive genotype primers | Negative genotype PCR |
|---|---|---|---|
| CTCF | GAAAGACTTACCAGAGACGC (SEQ ID NO: 12) | F: | |
| | | GCCTAATTCATTCACCAAAGGG (SEQ ID NO: 13) | |
| | | R: GGTGTGGCATATCATGGGTT (SEQ ID NO: 14) | |
| PAF1 | GGTGATGAACTTGGGGTCGA (SEQ ID NO: 15) | F:TTCTTCTCCACTAGGCCCAA (SEQ ID NO: 16) | |
| | | R: CTCTGGGGAGGAACTCAGAA (SEQ ID NO: 17) | |
| *SNCA* En-1 | 1: GTGCTACTATGATTCACTGA (SEQ ID NO: 18) | F: CAAACTCACAGCGTAGACAA (SEQ ID NO: 20) | F: |
| | | | CAAACTCACAGCGTAGACAA (SEQ ID NO: 20) |
| | 2: | R: AATTTCCTGGATGCTCAGTG (SEQ ID NO: 21) | |
| | GACGTGTGAAGTGATATATG (SEQ ID NO: 19) | | R: |
| | | | AATTTCCTGGATGCTCAGTG (SEQ ID NO: 21) |
| *SNCA* En-2 | 1: | F: TTGCATTTGCGTATTTGTGC (SEQ ID NO: 23) | F: |
| | GCCATATTGTGTAGCAACAG (SEQ ID NO: 5) | | GGGAATATTGGTGAGCATGG (SEQ ID NO: 25) |
| | | R: ACTTGGCTGAATCTGAGTCC (SEQ ID NO: 24) | |
| | 2: TCTTGATAGCTTCTAAGTCA (SEQ ID NO: 22) | | R: |
| | | | GGATCTAAGGACCCTCAACA (SEQ ID NO: 26) |

### 1.7 CRISPR/Cas9 knock-out SNCA En-1 and En-2

SNCA En-1 and En-2 knock-out was performed using CRISPR/Cas9 system. Two gRNA targets flanking SNCA Enhancer 1 (En-1) (Table 1, *SNCA* En-1 gRNA 1 and gRNA 2) or *SNCA* Enhancer 2 (SNCA En-2) (Table 1, *SNCA* En-2 gRNA 1 and gRNA 2) were designed by CRISpick (https://portals.broadinstitute.org/gppx/crispick/public) and subcloned into pX459 vector. Two paired gRNAs and pBabe were co-transfect (ratio, 1:1:0.2) into SK-N-AS or SH-SY5Y cells to knock-out *SNCA* En-1 or En-2. Twenty-four hours after transfection, cells were selected by puromycin (final concentration 2µg/ ml) for another 48 hours. The surviving cells were sub-cultured on 10 cm dishes until single clone formation. Genotype of single clone was done by PCR. Positive and negative genotype PCR primers were listed in Table 2.

**Table 2: List of all antibodies used in current study**

| Target | Source s | Company | Clone / Catalog Nr. | Previously Validation * | Current validation methods |
|---|---|---|---|---|---|
| THAP1 | Rabbit | Proteintech | 12584-1-AP | Publications | Knock-down / overexpression |
| Flag | Mouse | Sigma | F1804 | Publications | Overexpression |
| CEBPD | Mouse | Santa Cruz Biotechnology | C-6 | Publications | Overexpression |
| CTCF | Rabbit | Cell signaling | D31H2 | Publications | Gene knock-out |
| H3K27ac | Rabbit | Cell signaling | D5E4 | Publications | |
| H3K4me3 | Rabbit | Cell signaling | C42D8 | Publications | |
| TH | Rabbit | Millipore | 657012 | Publications | |
| Total SNCA | Mouse | BD Biosciences | 610786 | Publications | |
| Human SNCA | Mouse | Cell signaling | Syn204 | Publications | Transgenic model |
| Rat SNCA | Rabbit | Cell signaling | D37A6 | Publications | |
| UBTF | Mouse | Santa Cruz Biotechnology | F-9 | Publications | |
| PAF1 | Rabbit | Cell signaling | D9G9X | Manufacturer | Gene knock-out |
| WDR61 | Rabbit | Proteintech | 22536-1-AP | Manufacturer | |
| LEO1 | Rabbit | Proteintech | 12281-1-AP | Manufacturer | |
| CTR9 | Rabbit | Cell signaling | D1Z4F | Publications | |
| Rpb1 CTD | Mouse | Cell signaling | 4H8 | Publications | |
| Phospho-Rpb1 CTD (Ser5) | Rabbit | Cell signaling | D9N51 | Publications | |
| P300 | Rabbit | Cell signaling | E8S2V | Publications | |
| NELFE | Rabbit | Proteintech | 10705-1-AP | Publications | |
| Beta-Actin | Mouse | Cell signaling | 4967 | Publications | |
| Normal Rabbit IgG | Rabbit | Cell signaling | 2729 | Publications | |
| Mouse IgG Isotype | Mouse | Invitrogen | 31903 | Publications | |

### 1.8 Animal studies

All animal (rat) experiments were approved by the local Ethics Review Committees (Regierungspräsidium Tübingen). All the animal studies were performed according to "3R" principle (Replace, Reduce, and Refine). For protein analysis, RNA analysis, and immunohistochemistry staining, at least 3-5 animals (3-month-old) of each group were used for analysis. All experiments, including protein analysis, RNA-seq, ChIP-seq, were performed using three-month-old male rats. The following animal models were generated for this study:

### Human THAP1 transgenic rat model (THAP1 tg)

To generate human THAP1 gene transgenic rat model, whole *THAP1* gene (12 kb) were amplified from human genomic DNA using the following PCR primers (forward primer: CCTCATGAATGGCTTTGAGT (SEQ ID NO: 27), reverse primer: AAGCCAAGTAGCAATTCAGG (SEQ ID NO: 28)). The PCR product was sub-cloned into pUC19 vector using Kpnl and Sall restriction enzymes. After validate the sequences of positive clone by sanger-sequencing, the THAP1 gene fragment were digested and purified. High quality of THAP1 DNA fragment were directly inject into the pronucleus of rat zygotes to generate human THAP1 transgenic rat model. The expression of human THAP1 was analyzed at both mRNA and protein level.

### Human SNCA and human THAP1 double transgenic rat model (hSNCA/hTHAP1 tg)

The hSNCA/hTHAP1 double transgenic rat model were generated by crossbreeding of previously published SNCA tg rats with the newly generated human THAP1 tg rats.

### Human SNCA intronic enhancer cluster knock-out (SNCA EnKO) rat model

The SNCA intronic enhancer cluster knock-out rat model was generated by using CRISPR/Cas9 system. Two gRNAs were designed targeting two ends of the large intronic clsters region (about 48.6 kb) which include the SNCA En-1 and SNCA En-2 regions (gRNA1: CTGATACCACCTGCCTGCAC (SEQ ID NO: 7); gRNA2: GCATGGTCACAACTTACTAA (SEQ ID NO: 8)). Briefly, two synthesized gRNAs together with single-stranded oligodeoxynucleotides (ssODN) template and cas9 protein were injected into the pronucleus of rat zygotes. The genotype was done by positive PCR and negative PCR analyses. Positive PCR was performed using primers target two ends of residuals after knocking-out (Forward primer: GGCCAAAATGAACTAAATGAACAGGG (SEQ ID NO: 29); Reverse primer: GAACTTGGCTGAATCTGAGTCCATTC (SEQ ID NO: 30)). The negative PCR was performed using primers targeting the sequencing upstream and downstream of gRNA2 site (Forward primer: CGTATATGAAGGCTTTAAGGGTGTGC (SEQ ID NO: 31); Reverse primer: GAACTTGGCTGAATCTGAGTCCATTC (SEQ ID NO: 30))

### 1.9 Western blot

Using of human brain tissue and iPSCs differentiated mDA neurons for protein analysis was approved by ethic committee of University Hospital Tuebingen, Tuebingen, Germany. Western blot was performed as previously described in the art. Briefly, culture cells were lysis in RIPA buffer (1% NP-40; 0.5% sodium DOC; 0.1% SDS; 150 mmol NaCl; 50 mmol Tris; pH 8.0m, 1× proteinase inhibitor). After measuring protein concentration by BCA kit (Thermofisher Scientific), protein sample were separated by SDS gel and blotted onto nitrocellular membrane (millipore). After incubated with primary antibody (working concentration of different antibodies were listed in Table 2) and secondary antibody (IRDye^{®} 800CW Goat anti-Rabbit IgG (H + L) or IRDye^{®} 680RD Goat antiMouse IgG (H + L); 1:15000 in concentration), blot membranes were visualized under the Odyssey^{®} Fc Imaging System (Li-Cor). Each Western blot was repeated at least three times.

### 1.10 Immunohistochemistry staining

Immunohistochemistry staining was performed as reported previously in the art. Brains of male rats at 3 months of age were fixed by transcardiac perfusion with 4% paraformaldehyde (PFA) in PBS, followed by post-fixation with the same fixatives overnight at 4 °C. The half brain was imbedded in paraffin and subjected to microtome sectioning. DAB staining was utilized to staining the human SNCA and rat SNCA in SNCA tg rats and SNCA EnKO rats. In detail, the whole procedure was carried out at room temperature. The endogenous peroxidase activity of brain sections was blocked by using 0.5% sodium borohydride, followed by section permeabilization in TBS containing 0.4% Triton X-100 (TBST). The incubation in primary antibody anti-SNCA (anti-human SNCA 1:250; anti-rat SNCA 1:500) was performed overnight followed by secondary antibody staining using biotinylated goat anti-rabbit at a dilution of 1:1000 (Vector Laboratories). Then sections were treated with an avidin-biotin complex (Vector Laboratories, BA-1000), and exposed to DAB-H₂O₂ (0.01% DAB, and 0.001% hydrogen peroxide) until a suitable staining intensity had developed.

### 1.11 Luciferase reporter assay

Luciferase reporter assays were performed as previously described in the art. The full SNCA promoter region (10.7 kb) was amplified by PCR and sub-cloned into pGL3 empty vector. Activities of firefly and Renilla luciferase was measured at 48 hours after co-transfection of pGL3 vector with pcDNA/THAP1 or pcDNA/Empty vector by incubating with the Dual Luciferase Reporter Assay System (Promega) in a Mithras-Luminometer (Berthold Technologies). *TOR1A* promoter was used as positive control as THAP1 can repress the activity of TOR1A promoter. All experiments were verified at least in five independent replicates.

To detect the interactions between *SNCA* enhancers and promoter, the core promoter region (1.34kb) of *SNCA* gene was amplified from the whole *SNCA* promoter using the following primers (Forwards primer: AGCTAGCAAAGGCTTTCTGCTA (SEQ ID NO: 32); Reverse primer: TCTCGAGGGTGGAAAGGCAGAA (SEQ ID NO: 33)) and sub-cloned into pGL3 basic vector (Promega) using Nhel and Xhol restriction enzyme. The *SNCA* En-1 region (1560 bp) (Forward primer: CAAACTCACAGCGTAGACAA (SEQ ID NO: 20); Reverse primer: AATTTCCTGGATGCTCAGTG (SEQ ID NO: 21)), SNCA En-2 region (1496 bp) (Forward primer: TAAAGGGGACTGGGAAAGTT (SEQ ID NO: 34); Reverse primer: AAAACCATCACCACAGTTCC (SEQ ID NO: 35)), and negative control region (1504 bp) (Forward primer: AATGCACTGGAAAGGTTGTT (SEQ ID NO: 36); Reverse primer: TCTTTTTCCCCATGAGGTCT (SEQ ID NO: 37)) were amplified from human genomic DNA and sub-cloned into pGL3/SNCA core promoter vector, respectively. The sub-cloned pGL3 vector carrying both *SNCA* promoter and enhancer were co-transfected with pRL vector (Promega) into SH-SY5Y cells to analyze the interaction between *SNCA* promoter and enhancer.

### 1.12 Nuclear protein extraction

Nuclear proteins extracted from HEK cells, SH-SY5Y cells, or brain tissues were used for analyzing of THAP1 protein level or for Tandem Affinity Purification and Mass Spectrometry (TAP-MS) experiments. Briefly, the cytoplasmic fraction was collected using buffer A (10 mM HEPES (Sigma), 1 mM EDTA (Applichem), 0.1 mM EGTA (Sigma), 10 mM KCI (Carl-Roth), 1 mM PMSF (Sigma), 1 µg/ml phosphatase inhibitor cocktail 2 and 3 (Sigma), 1mM DTT (Merck), 1 µg/ml complete protease inhibitor (Roche)). After 15 min of incubation, the NP40 (Roche) was added to the final concentration of 0.1% in order to break the cell membrane. Following centrifugation at 10000xg for 5min at 4°C the supernantant was removed (cytoplasmic fraction) and the nuclei were harshly re-suspended in buffer C (20 mM HEPES, 0.2 mM EDTA, 0.1 mM EGTA, 25% glycerol (Carl-Roth), 420 mM NaCl (Merck), 1.5 mM MgCl2 (Sigma), 1 mM PMSF, 1 µg/ml phosphatase inhibitor cocktail 2 and 3, 1 mM DTT, 1 µg/ml complete protease inhibitor) and rotated on an intellimixer for 20 min at 4°C. After centrifugation at 10000xg for 5min at 4°C the supernatant containing nuclear proteins was collected.

### 1.13 Tandem Affinity Purification and Mass Spectrometry (TAP-MS)

TAP-MS was performed as reported previously in the art. TAP was performed in HEK-293 cells overexpression Strep/FLAG tagged THAP1 protein. Nuclear protein were extracted as described before and incubated with anti-Flag M2 agarose affinity gel (Sigma). During the incubation, 300U benzonase nuclease (Sigma) per mg nuclear extract was added to disturb the DNA- or RNA- mediated interactions. The eluted protein solution by flag peptide was treated by trypsin for quantitative MS/MS analysis (LC-MS/MS). Raw data were quantified by building ratios of the peak area intensities from specific fragment ions of the corresponding samples. Each group (Empty vector group and wild-type THAP1 group) were repeated five times.

### 1.14 Chromatin immunoprecipitation and sequencing (ChIP-seq)

ChIP sample preparation and high through-put sequncing were done as previously described in the art. For brain tissues, frozen brain tissues were thaw and cut into less than 1 mm³ pieces. PBS with 1% formaldehyde (ThermoFisher scientific) was added to crosslink tissues for 15 minus. After added Glycine and incubated for another 5 minutes, tissues were collected by centrifuging. For cell samples, cells were crosslinked by directly adding formaldehyde into the culture medium to the final concentration of 1% and incubated for 10 min at room temperature. After adding Glycine to the final concentration of 0.125M and incubating for 5 minus, cells were collected. Cell pellet / tissue pellet were sonicated in RIPA buffer (10 mM Tris-HCl, 1 mM EDTA, 0.5% sodium deoxycholate, 0.1% SDS, 1% NP-40, 1× Protease inhibitor cocktail, pH 8.0) to 200-500 bp using Covaris S220. After centrifugation, chromatin were incubated with antibody-beads complex, which was prepared one day before by incubating antibody with goat anti-Rabbit IgG / Mouse IgG magnetic beads (ThermoFisher scientific) overnight. After incubation the chromatin and antibody-beads complexes overnight, the complexes were washed by low salty buffer, high salty buffer, LiCI buffer, and TE buffer. The ChIP DNA-protein complex was eluted from beads and de-crosslinked overnight. Finally, ChIP DNA was extracted using PCR purification kit (QIAGEN). At least 5ng of immunoprecipitated DNA was used for the libraries preparation using the NEB Ultra II DNA Library Preparation Kit for Illumina (NEB, E7103L). Next generation sequencing was done on Illumina NextSeq500 / NovaSeq 6000 system using the 75bp / 100bp single-end high output sequencing kit.

### 1.15 ChIP-qPCR

Quantitative analysis of ChIP results was performed using ChIP-qPCR. The ChIP-qPCR was performed using QuantiTect PCR Kits (QIAGEN) as previously described in the art. The ChIP input sample was used to calculate ChIP binding signal (as Input %). IgG ChIP sample was used as negative control. Each experiment was repeated at least three times. Primers used for ChIP-qPCR were listed in the Table 3. To analyze the RNA Polymerase II pause, *SNCA* promoter primers were used to detect the Pol II binding on promoter region, the *SNCA* Exon 2 and *SNCA* Exon 4 were used to detect the Pol II binding on SNCA gene body.

**Table 3: List of primers used for ChIP-Qpcr**

| Regions | Sequences | PCR product (bp) |
|---|---|---|
| *SNCA* promoter | F: GGAAAATGGTGATAGTGGCG (SEQ ID NO: 38); R: TGAGGGTGAAAGGGAGACTA (SEQ ID NO: 39) | 97 |
| *SNCA* En-1 | F: TTCATCTTCTCCAGGCACAG (SEQ ID NO: 40); R: GGTTACTTTTGTGGGGTGTG (SEQ ID NO: 41) | 87 |
| *SNCA* En-2 | F: AATAATTGCCGACTGTGACC (SEQ ID NO: 42); R: GTGATGGAGGGAAATCGTGT (SEQ ID NO: 43) | 96 |
| *SNCA* Exon 2 | F: GCTGAGAAAACCAAACAGGG (SEQ ID NO: 44); R: GAACAAGCACCAAACTGACA (SEQ ID NO: 45) | 103 |
| *SNCA* Exon 4 | F: GTCAAAAAGGACCAGTTGGG (SEQ ID NO: 46); R: CCAGGCCTCACATGAAAATG (SEQ ID NO: 47) | 96 |
| *SNCA* Control | F: GTCTATGCCCTGTTTAGCAATC (SEQ ID NO: 48); R: TCTTACCCTGTAGGACTTTCAT (SEQ ID NO: 49) | 96 |

### 1.16 mRNA-seq and total RNA-seq

Total RNA was isolated using RNeasy Mini Kit (QIAGEN) or RNeasy Lipid Tissue Mini Kit (QIAGEN) following manufacturer's protocol. RNA samples used for library preparation presented a high integrity number (RIN > 9). For mRNA-seq, a total of 100ng of total RNA was subjected to polyA enrichment, and cDNA libraries were constructed using the resulting mRNA and the NEBNext Ultra II Directional RNA library Preparation Kit for Illumina (NEB, #E7760L). For total RNA-seq, a total of 100 ng total RNA was digested by ribosome RNA and subjected to libraries preparation using NEBNext Ultra II Directional RNA library Preparation Kit for Illumina (NEB, #E7760L). Libraries were sequenced as paired-end 100bp reads on a NovaSeq6000 (Illumina) with a depth of approximately 20 million reads each. Library preparation and sequencing procedures were performed by the same individual aimed to minimize technical batch effects was chosen.

### 1.17 Reverse-transcript PCR

The expression level of SNCA mRNA in SNCA EnKO rats were analyzed by reverse transcript PCR (RT-PCR) and quantitative PCR (RT-qPCR) as previously described (Cheng et al., 2012). In brief, brain tissue total RNA were extract using RNeasy Lipid Tissue Mini Kit (QIAGENE) and reverse transcript into cDNA using Omniscript RT Kit. Total human SNCA cDNA transcript was amplified using primers targeting exon 2 and 3' untranslated region (UTR) of SNCA gene (Forward primer: AAGCAGCAGGAAAGACAAAA (SEQ ID NO: 50); Reverse primer: ACATCTGTCAGCAGATCTCA (SEQ ID NO: 51)). Quantitative RT-PCR of the human SNCA mRNA were performed using QuantiTect PCR Kits (QIAGEN) with the primers targeting exon 5 and 3' UTR of SNCA gene (Forward primer: CTGTGGATCCTGACAATGAG (SEQ ID NO: 52); Reverse primer: CAAGAAACTGGGAGCAAAGA (SEQ ID NO: 53)). Rat housekeeping gene actin was used as internal control (Forward primer: AGAAGGAGATTACTGCCCTG (SEQ ID NO: 54); Reverse primer: CCACCAATCCACACAGAGTA (SEQ ID NO: 55)).

### 1.18 4C-seq

Circular chromatin conformation capture with high-throughput sequencing (4C-seq) was performed as previously described in the art with the following modification: (1) Nlalll (New England Biolabs) and Bfal (ThermoFisher Scientific) were selected as the first and second restriction enzymes, respectively; (2) Before generating 4C-seq libraries for sequencing, 4C template of viewer point (VP: SNCA promoter) were amplified by PCR by 6 cycles using specific primers (Forward primer: ACGAATGGTCGTGGGCACCG (SEQ ID NO: 56); Revese primer: CCTCTCTTGGGCCCCTTCTG (SEQ ID NO: 57)). Final 4C-seq libraries were generated by PCR of 24 cycles using indexed primers (P5 and P7).

(Forward: AATGATACGGCGACCACCGAGATCTACACTCTTTCCCTACACGACGCTCTTCCGATC TNNNNNNACGAATGGTCGTGGGCACCG (SEQ ID NO: 58); Reverse: CAAGCAGAAGACGGCATACGAGATGCCGTCCTCCTCCTCCTAG (SEQ ID NO: 59)) (NNNNNN: Barcode sequences used for isolating reads of different samples). Next generation sequencing was done on Illumina NextSeq500 system using the 150bp single-end high output sequencing kit.

### 1.19 Data analysis

### ChIP-seq data analysis

ChlP-seq raw reads were aligned to the hg19 genome or rn6 genome assembly using Bowtie2. Only reads that uniquely mapped to the genome were used for further analysis. ChIP-seq peaks calling were analyzed using MACS2. Bam files were converted into coverage bigWig file using deeptools (Version 3.3.2) and samtools (Version 1.9) package and viewed using Integrative Genomics Viewer (IGV) browsers (version 2.12.2).

### RNA-seq data analysis

Read quality of RNA-seq data in fastq files was assessed using QoRTs (v1.2.37) to identify sequencing cycles with low average quality, adaptor contamination, or repetitive sequences from PCR amplification. Reads were aligned using HISAT2 (Version 2.2.1) allowing gaped alignments to account for splicing against a custom-built genome composed of the Ensembl Homo Sapiens GRCh37 and Alignment quality was analyzed using samtools (v1.1) and visually inspected in the Integrative Genome Viewer (Version 2.12.2). Normalized read counts for all genes were obtained using featureCount (v3.18.1). Bam files were converted into coverage bigWig file using deeptools (Version 3.3.2) and samtools (Version 1.9) package and viewed using Integrative Genomics Viewer (IGV) browsers (version 2.12.2).

### 4C-seq data analysis

Raw reads were separated into individual sample FastQ Files by barcode sequences. Raw reads of each sample were filtered by retaining VP-specific PR amplicons (GGAGGGGGTGGTGCTGC (SEQ ID NO: 60)), trimmed to remove nucleotide sequences belongs to the VP restriction fragment, and subsequently mapped to the hg19 genome assembly using Bowtie2. Only uniquely mapped reads were used to further analyses with Basic4C and 4C-ker packages. Mapped raw read were saved as bedgraph files and viewed using IGV browsers (version 2.12.2).

### 1.20 Statistical analyses

All the data presented in this study were analyzed using two-tailed unpaired t-test when two groups were compared. Statistical analyses were performed using the Prism software 10.0 (GraphPad Software, La Jolla, CA).). Significant differences were considered when *p ≤ 0.05; **p ≤ 0.01, ***p ≤ 0.001, and ****p ≤ 0.0001.

### 1.21 Data availability

The RNA-seq, ChIP-seq, and 4C-seq datasets generated in this study are available through the Gene Expression Omnibus (GEO) via accession GSE141278 and GSE184961.

### 2. Results

### THAP1 mutations repress the expression of SNCA

RNA-seq analysis of iPSC-derived mDA neurons from THAP1/DYT6 patients and THAP1 heterozygous knock-out (THAP1^{+/-}) dopaminergic SH-SY5Y neuronal cells showed significantly decreased expression of SNCA, indicating THAP1 as a potential transcriptional regulator of SNCA. Extended western blot analysis confirmed decreased SNCA protein level in these cells (Fig. 1A and 1B). In post-mortem frontal cortex samples of THAP1 patients, the inventors also observed significantly reduced SNCA protein level compared to brain tissue of neurologically normal deceased controls (Fig. 1C). Conversely, overexpression of THAP1 in both SH-SY5Y and SK-N-AS neuroblastoma cells lead to a significant increase of SNCA expression (Fig 2A). All these observations suggest that THAP1 may regulate the expression of SNCA.

### Human THAP1 regulates human and rat SNCA expression in brain of transgenic rats

In order to further prove the role of THAP1 in regulating SNCA expression in brain, the inventors generated THAP1 overexpressing transgenic rats (THAP1 tg) by pronuclear injection of the entire human THAP1 gene (12 kb) (Fig. 2B). Both reverse transcript PCR (RT-PCR) and western blot analysis confirmed the expression of wild-type human THAP1 (hTHAP1) in different brain regions of THAP1 tg rats (Fig. 1D and Fig. 2C). The highest expression of hTHAP1 was seen in cerebellum, similar to the expression patterns of endogenous rat THAP1 protein (rTHAP1) (Fig. 1D). The inventors also observed that the molecular weight of hTHAP1 protein is slightly smaller than rTHAP1 protein (Fig. 1D), although the predicted molecular weight of hTHAP1 is 24.944 kDa, which is slightly larger than the predicted molecular weight of rTHAP1 (24.688 kDa)
(https://www.uniprot.org). These data may indicate different posttranslational modifications of these two proteins, like methylation.

The inventors next crossbred the THAP1 tg rats with our previously generated human SNCA transgenic rats (SNCA tg). Significantly increased expression of both rat SNCA (rSNCA) and human SNCA (hSNCA) were detected in cerebellum of hSNCA/ hTHAP1 double transgenic rats (SNCA tg/ THAP1 tg) compared to SNCA tg rats (Fig. 1E). Increased expression of hSNCA and rSNCA were also seen in striatum and in cortex, but their changes were weaker than in cerebellum (Fig. 1E, Fig. 2D and 2E). The expression change of hSNCA and rSNCA are correlated to the expression level of human THAP1, as cerebellum expresses more hTHAP1 protein than striatum and cortex (Fig. 1D). These data further confirm the role of THAP1 in regulating the expression SNCA in brain *in vivo.*

When comparing the SNCA tg rats with non-transgenic rats (Non-tg), the inventors observed decreased rSNCA protein level (Fig. 1E, Fig. 2D and 2E), which may suggest that overexpression of hSNCA protein represses the expression of endogenous rSNCA. The inventors next analyzed the expression of rSNCA, hSNCA, and total SNCA (tSNCA) in two different SNCA transgenic rat lines, the SNCA tg line which expresses very high level of human SNCA protein and the SNCA tg-L line which expresses very low level of human SNCA protein, and observed that the rSNCA protein was decreased only in the SNCA tg line but not in SNCA tg-L line (Figure 1F, Figure 2F). These data suggest the autoregulative function of SNCA, but it may happen only when the human SNCA protein reached a certain expression level.

### THAP1 regulates the activities of SNCA promoter and intronic enhancers

In order to understand how THAP1 regulates SNCA expression, we examined our THAP1 chromatin immunoprecipitation sequencing (ChIP-seq) data and observed weak binding signals to the SNCA promoter (Figure 3A). Further luciferase reporter assay analysis confirmed that wild-type THAP1 can up-regulate the activity of the entire SNCA promoter [10.7 kb upstream of translation start site (Figure 4A).

H3K27ac and H3K4me3 ChIP analyses were further performed to see the activities of all SNCA regulatory elements. As the THAP1^{+/-} SH-Y5Y cell lines still express close to normal levels of wild-type THAP1 protein due to its auto-regulative function, we generated THAP1 knock-down SH-SY5Y cell lines (shTHAP1) in which the THAP1 protein was reduced to 30% (Figure 4B). Similar decreased expression of SNCA was also observed in the shTHAP1 cell lines (Figure 4C). Significantly decreased H3K27ac modification on both, SNCA promoter and potential enhancers, including enhancer 1 (En-1) and enhancer 2 (En-2), were seen in shTHAP1 cell lines compared to control cell lines (shControl) (Fig. 3A). The changes of SNCA enhancers' activities are stronger than changes of its promoter (Figure 4D). Similar decreased H3K27ac and H3K4me3 modifications on SNCA promoters and enhancers were also seen in THAP1 patients' iPSC-derived mDA neurons compared to control iPSC-derived mDA neurons, respectively (Fig. 3B).

As THAP1 has no direct binding site on SNCA enhancers (Fig. 3A), its regulation on the SNCA enhancers' activities might be through the transcriptional repressor of SNCA, the CEBPD (CCAAT/enhancer-binding protein delta), as both RNA-seq and western blot analyses showed highly increased expression of CEBPD in THAP1 patients' iPSC-derived mDA neurons compared to control neurons (Fig. 3C). The following data further supports this hypothesis: (1) The expression level of SNCA is negatively correlated with the expression level of CEBPD in iPSC-derived mDA neurons (Fig. 3D); (2) THAP1 binds to the *CEBPD* promoter (Figure 4E), and CEBPD binds to the *SNCA* promoter and enhancers (Fig. 3A); (3) Knock-down of THAP1 induced the expression of CEBPD and strengthen the binding activities of CEBPD on SNCA promoter (Figure 4F and 4G); and (4) overexpression of CEBPD indeed repressed the expression of endogenous SNCA in SH-SY5Y cells (Figure 3E).

### THAP1 interaction partner CTCF regulates SNCA expression

Next, the inventors want to know whether THAP1's interaction partners are also the transcription regulators of SNCA. The inventors thus performed Tandem Affinity Purification and Mass Spectrometry (TAP-MS) analysis to isolate the interaction partners of THAP1. In TAP-MS, we overexpressed flag-tagged THAP1 protein in HEK cells and pulled-down THAP1 protein and its interaction partners using anti-flag agarose gel. Finally, Mass Spectrometry analysis was performed to identify all these proteins. Using TAP-MS analysis we isolated a total of 149 proteins which strongly interact with THAP1 (with log2 > 15) (Fig. 5A). String Protein-Protein Interaction Networks analysis grouped these proteins into five functional complexes (Fig 5B), like the RNA polymerase II associated factor 1 (PAF1) complex (PAF1C) and the chromatin assembling complex, including CTCF (CCCTC-binding factor / Transcriptional repressor CTCF) (Fig. 5B), which have potential function in regulating messenger RNA expression. Western blot analysis confirmed the interactions between THAP1 and its interaction partners, like CTCF (Figure 6A). ChlP-seq showed that CTCF binds to the SNCA promoter and a region near the SNCA En-2 (Fig. 3A). Knocking-out CTCF down-regulates the expression of SNCA, while overexpression of CTCF leads to up-regulation of SNCA (Fig. 5C, Figure 6B). The inventors also knocked-out PAF1 in SH-SY5Y, a key component of PAF1C, but did not observe any expression change of SNCA (Fig. 5D and Figure 6C). These data suggest that THAP1's interaction partner CTCF but not PAF1 is a transcription regulator of *SNCA* gene.

### SNCA enhancers in intron 4 contact with its promoter and regulate its expression

As THAP1 regulates SNCA expression through controlling its enhancers and promoter activities, the inventors next wanted to know whether these enhancers indeed contact physically with the SNCA promoter and regulate its activities. The inventors thus performed Circular Chromosome Conformation Capture followed by sequencing (4C-seq) analysis in which the *SNCA* promoter was set as view point (VP). The inventors bserved strong physical contact between SNCA En-2 region and its promoter (Fig. 7A and 7B). Other regions, like *SNCA* En-1 and *MMRN1* promoter, also showed contact with the *SNCA* promoter when analyzing the 4C-seq data with Basic4C package (Fig. 7B). As CTCF binds to both, *SNCA* promoter and *SNCA* enhancer En-2 (Fig. 7A), and as it does function in regulating enhancer-promoter interactions, the inventors investigated whether it also mediated the SNCA enhancer-promoter interaction. Indeed, knocking-out CTCF reduced the contact frequencies between the VP (*SNCA* promoter) and several loci located on the intron 4 of the *SNCA* gene (Fig. 7A and 7C). Thus, CTCF regulates the structure of *SNCA* enhancer-promoter loops.

In order to further validate the function of *SNCA* intronic enhancers, the inventors subcloned the SNCA En-1 and En-2 fragments into the C-terminal of the *luc*+ gene in pGL3 basic vector, respectively, in which the expression of *luc*+ gene was driven by the SNCA core promoter (Fig. 7D). Compared to genomic unrelated fragment (control), both SNCA En-1 and En-2 fragments can up-regulate the activity of SNCA promoter, while the fragment En-2 has stronger function than the En-1 fragment (Fig. 7D). Next, the inventors knocked-out *SNCA* En-1 and En-2 in SH-SY5Y cells, respectively, to see how much these enhancers control the expression of SNCA (Figure 8A and 8B). Knocking-out of *SNCA* En-1 significantly reduced its expression by about 20%, while knocking-out En-2 drastically repressed 75% of its expression (Fig. 7E and 7F). We also knocked-out the SNCA En-1 in SK-N-AS neuroblastoma cell line, in which the SNCA En-1 is inactivated (Supplementary figure 4C). Unexpectedly, we did not observe any expression changes of SNCA after knock-out its En-1 region in this cell line (Figure 8C). These data suggest that both SNCA enhancers can regulate its expression, but only when they are activated. The *SNCA* En-2 plays predominant function in controlling the expression of SNCA in SH-SY5Y cells.

### Lineage-specific SNCA enhancers control its expression during neurodevelopment and are related to PD risk

Next, the inventors sought to know whether the activation of the human SNCA enhancer is associated to its expression in neurons and in brain. The inventors first analyzed the activation of these enhancers during neuronal differentiation of iPSCs by using H3K27ac ChlP-seq analysis. The inventors observed that the activities of SNCA En-2 present cell lineage-dependent changes (Fig. 9A). For example, it is inactivated in iPSCs and iPSC derived neural progenitor cells (iPSC-NPCs) but moderately activated in iPSCs derived mDA neurons (iPSC-Neurons). However, in the cortex and cerebellum of SNCA tg rats, this intronic enhancer region is highly activated and expanded over 30 kb to form enhancer clusters (defined as Intra-Cluster), like a "super-enhancers" (Fig. 9A, Figure 10A). Further ChIP analysis revealed the enrichment of transcription factors P300 in this region (Figure 10B), and total RNA-seq analysis of human frontal cortex detected the expression of enhancer RNA (eRNA) in this region (Figure 10A), which data further support the characteristics of this intronic enhancer clusters as super-enhancers. Similar enhancer clusters on rat *Snca* gene were observed in human and rat brain, respectively (Figure 10D). Furthermore, the region down-stream of the SNCA gene also showed cell lineage-dependent changes and formed enhancer clusters in brain (defined as Inter-Cluster in Fig. 9A).

In parallel with the activation of *SNCA* enhancers, its expression level increased correspondingly (Fig. 9B). Very low levels of SNCA were observed in iPSCs and iPSC-NPCs, while moderate SNCA expression was seen in iPSC-Neurons. Because the expression levels of human SNCA in different SNCA transgenic rat lines are varied (Fig. 1F), we thus analyzed the expression of SNCA mRNA in human brain. Extremely high expression levels of SNCA were observed in human cerebellum and frontal cortex (Fig. 9B). These data further suggest that the activities of *SNCA* enhancers may be responsible for the elevated expression of SNCA in neurons and in brain. Genome-wide association study (GWAS) data meta-analysis revealed that the SNPs with highest association with PD risk (-Log10 (P-value) > 30) are enriched in the enhancer cluster regions of *SNCA* (the Intra-cluster and Inter-cluster in Fig. 9A bottom). As these enhancer regions can regulate SNCA expression, the PD risk associated SNPs within these regions may affect the enhancer activities and SNCA expression which thus contribute to sporadic PD risk.

### SNCA intronic enhancer cluster predominantly control the expression of SNCA in brain

Next, the inventors want to know whether the intronic enhancer clusters also predominantly regulates the expression of SNCA in brain, as the SNCA En-2 region significantly controls SNCA expression in dopaminergic SH-SY5Y cells (Fig. 7E and 7F). For this purpose, the inventors knocked-out this intronic enhancer clusters (Intra-Cluster: a region includes *SNCA* En-1 to En-2) of the human *SNCA* gene in our SNCA tg-L rats (Fig. 11A). In order to exclude the copy number changes / re-arrangement during enhancer knock-out, the inventors analyzed the copy number of the human SNCA gene in our two different human SNCA transgenic rat lines (Figure 1F) using whole genome sequencing data (with the same sequencing depth). The inventors observed that the copy number of the human *SNCA* gene in SNCA tg rats is ten times more than the copy number in SNCA tg-L rats (Figure 12A). The inventors also observed that the SNCA tg-L rats harbor only one copy of the human SNCA gene, as the reads intensity of the rat Snca gene (2 copies) is two times more than the intensity of the human *SNCA* gene in the same rat (Figure 12B). Utilizing the CRISPR/Cas9 technique, the inventors generated a SNCA EnKO rat model by knocking-out the intronic enhancer clusters in the SNCA tg-L rats (Fig. 11A, Figure 12C). Although whole genome sequencing and H3K27ac ChlP-seq both detected the deleted intronic region fragment in SNCA EnKO rat genome (Figure 12C), this DNA fragment has been cut from human SNCA gene and might be inserted into other genomic regions of the rat genome, as both the whole genome sequencing and sanger-sequencing confirmed the DNA break point at the gRNA target sites and re-ligation of two residual ends in the SNCA EnKO rats (Figure 12C).

The inventors next tested both rSNCA and hSNCA expression in the SNCA EnKO rat. Unexpectedly, both western blot analysis and immunohistochemistry staining did not detect hSNCA protein in the entire brain of SNCA EnKO rats (Fig. 11B and 11C). As both methods are not sensitive to detect extremely low levels of target protein, the inventors thus analyze the mRNA level of human SNCA in the SNCA EnKO rat model using reverse-transcript PCR (RT-PCR) and quantitative PCR (RT-qPCR). Both methods detected extremely low level of human SNCA mRNA in SNCA EnKO rat compared to SNCA tg-L rats (1.49%) (Fig. 11D and 11E). Confirming Sanger sequencing excluded mutations, aberrant splicing, or exon skipping of the human SNCA mRNA in SNCA EnKO rat (Figure 12D). These data suggest that knocking-out the intronic enhancer clusters drastically reduces the expression of human SNCA in the brain.

### SNCA intronic enhancer cluster regulates its expression via releasing of paused RNA Polymerase II

Enhancer induces gene expression could be through promoting transcription initiation, release of paused Polymerase II (Pol II), or productive elongation. In order to see how the SNCA intronic enhancer clusters control its expression in brain, the inventors next performed H3K27ac and H3K4me3 ChlP-seq and ChIP-qPCR to check the human SNCA promoter activities in SNCA EnKO rats. Deletion of the intronic enhancer clusters leads to around 50% reduction of the H3K27ac and H3K4me3 modifications on the human SNCA promoter (Fig. 11F and 11G). However, the change of promoter activity did not match to the change of mRNA expression, because the human SNCA mRNA decreased to 1.49% after knocking-out the intronic enhancer cluster (Fig. 11E). The inventors thus performed Polymerase II (Pol II) and S5 phosphorylated Polymerase II (P5-Pol II) ChIP-qPCR to examine the transcription initiation of human SNCA gene in cortex and observed slightly increased Pol II and P5-Pol II binding activities on the SNCA promoter in SNCA EnKO rat cortex (Fig. 13A and 13B), which suggest higher transcription initiation of *SNCA* gene in SNCA EnKO rats. Next, the inventors compared the Pol II binding signals on *SNCA* promoter and gene body, like exon 2 and exon 4, between SNCA tg-L rat cortex and SNCA EnKO rat cortex to see the RNA Pol II pausing release. The inventors observed stronger binding activities of Pol II on exon 2 and exon 4 of the human *SNCA* gene in SNCA tg-L rat cortex than in SNCA EnKO rat cortex (Fig. 13A). Remarkably, the enrichment of Pol II over exon 4 in the EnKO rat cortex became barely detectable in comparison to a non-transcribed genomic region (control) (Fig. 13A). Further ChIP-qPCR analysis of negative elongation factor E (NELFE), an essential component of the negative elongation factor (NELF) complex, revealed stronger binding activities on SNCA promoter in SNCA EnKO rat cortex compared to in SNCA tg-L rat cortex (Fig. 13C). These data strongly support that in the SNCA EnKO rat brain the RNA polymerase II is paused on SNCA promoter, leading to suppressed transcription elongation (Fig. 13D), which is responsible for the drastic reduction of SNCA expression. Thus, the *SNCA* intronic enhancer clusters predominantly regulate the expression of SNCA through promoting the release of paused RNA polymerase II and transcription elongation.

### 3. Conclusion

The inventors provide for the very first time tools to modify the expression of the *SNCA* gene specifically in the brain of a subject and, thereby, an approach for the therapeutic treatment of PD and other SNCA-associated disorders, which overcomes the disadvantages of the concepts so far described in the art.

## Claims

1. A composition for modification of the expression of a *SNCA* gene, the composition comprising:
(a) at least one nucleic acid molecule comprising a nucleotide sequence substantially complementary to the nucleotide sequence of a target region within an intronic enhancer of the *SNCA* gene located within intron 4.

2. The composition of claim 1, wherein said at least one nucleic acid molecule is
(i) a guide RNA (gRNA), or/and
(ii) a nucleic acid molecule encoding said at least one gRNA.

3. The composition of claim 2 further comprising:
(b)
(i) a Clustered Regulatory Interspaced Short Palindomic Repeats associated (Cas) protein, or/and
(ii) a nucleic acid molecule encoding said Cas protein,
wherein said gRNA is configured to guide the Cas protein to a target region within an enhancer of the *SNCA* gene.

4. The composition of claim 1 wherein said at least one nucleic acid molecule is an antisense oligonucleotide (ASO) against enhancer RNA (eRNA).

5. The composition of any of the preceding claims, wherein the *SNCA* gene is a human *SNCA* gene (*hSNCA*), preferably
the enhancer is located at Chr4: 90720577-90722136 (En-1) and/or at Chr4: 90673800-90675200 (En-2), further preferably
the enhancer comprises the nucleotide sequence of SEQ ID NO: 1 (En-1) and/or SEQ ID NO: 2 (En-2).

6. The composition of any of the preceding claims, wherein the at least one gRNA comprises a nucleotide sequence of at least one of SEQ ID NO: 3 (gRNA1F), SEQ ID NO: 4 (gRNA1R), SEQ ID NO: 5 (gRNA2F), SEQ ID NO: 6 (gRNA2R), SEQ ID NO: 7 (gRNAtF), SEQ ID NO: 8 (gRNAtR), preferably
at least one pair of gRNAs is provided [(a)(i)] or encoded [(a)(ii)] as follows:
- SEQ ID NO: 3 (gRNA1F), SEQ ID NO: 4 (gRNA1R), and/or
- SEQ ID NO: 5 (gRNA2F), SEQ ID NO: 6 (gRNA2R), and/or
- SEQ ID NO: 7 (gRNAtF), SEQ ID NO: 8 (gRNAtR).

7. The composition of any of the preceding claims, wherein the Cas protein is a Cas9 protein, preferably a SpCas9 protein, further preferably the Cas protein is a dead Cas9 (dCas9) protein, further preferably a dCas9 fused to a protein domain exhibiting histone-deacetylation activity, and highly preferably dCas9 fused to an HDAC3 protein and/or a peptide derived from the Krüppel associated box (KRAB) domain.

8. The composition of any of the preceding claims wherein the nucleic acid of (a)(ii) and/or (b)(ii) comprises DNA or RNA.

9. The composition of any of the preceding claims, wherein one or both of (a) and (b) are packaged in a vector, preferably a viral vector.

10. The composition of any of the preceding claims for use in the treatment of a neurological disease, preferably a neurodegenerative disease, further preferably selected form the group consisting of: synucleopathies, including α-synucleopathy, *SNCA*-associated neurodegenerative diseases, multiple system atrophy 1 (MSA1), Parkinson's disease, and dementia with Lewy bodies.

11. An isolated polynucleotide encoding the composition of any of the preceding claims.

12. A vector, preferably a viral vector, comprising the isolated polynucleotide of claim 11.

13. A pharmaceutical composition comprising at least one of the composition of claims 1-10, the isolated polynucleotide of claim 11, the vector of claim 12, or combinations thereof.

14. A kit comprising at least one of the composition of claims 1-10, the isolated polynucleotide of claim 11, the vector of claim 12, or combinations thereof.

15. A method of modulation of expression of the *SNCA* gene in a cell, the method comprising contacting the cell with at least one of the composition of claims 1-10, the isolated polynucleotide of claim 11, the vector of claim 12, the pharmaceutical composition of claim 13, or combinations thereof, in an amount sufficient to modulate the expression of the *SNCA* gene.
